# EUROPEAN PATENT SPECIFICATION

(11) **EP 1 084 259 B1**
(45) Date of publication and mention of the grant of the patent: **28.05.2008**
(21) Application number: 99927888.0
(22) Date of filing: 04.06.1999
(51) Int. Cl.: C12N 15/57, C12N 9/64, C12N 5/10, C12Q 1/37, C07K 16/40

(54) **CORIN, A SERINE PROTEASE**
CORIN, EINE SERINPROTEASE
LA CORINE, UNE SERINE PROTEASE

(30) Priority: 05.06.1998 US 92029; 20.05.1999 US 314967
(43) Date of publication of application: 21.03.2001
(73) Proprietor: Bayer Schering Pharma Aktiengesellschaft, 13353 Berlin (DE)
(72) Inventor: MORSER, Michael, John, San Francisco, CA 94114 (US); WU, Qingyu, El Sorbante, CA 94803 (US); YAN, Wei, Hercules, CA 94547 (US)
(74) Representative: Pohlman, Sandra M.
(86) International application number: PCT/EP1999/003895
(87) International publication number: WO 1999/064608

(56) References cited:
- WO-A-98/03665
- WO-A-98/36054
- WO-A-98/45436
- EMBL/GENBANK DATABASES Accession no AA147031 Sequence reference HSAA47031 14 December 1996 HILLIER L ET AL: "The WashU- Merck EST project" XP002119681 cited in the application
- EMBL/GENBANK DATABASES Accession no AA906367 Sequence reference AA906367 9 April 1998 "National Cancer Insitute, Cancer Genome Anatomy Project" XP002119682
- EMBL/GENBANK DATABASES Accession no AA249210 Sequence reference HS1163286 LIEW C: "cDNAs from human fetal heart" XP002119683
- TOMITA Y ET AL: "A novel low-density lipoprotein receptor-related protein with type IImembrane protein-like structure is abundant in heart" JOURNAL OF BIOCHEMISTRY., vol. 124, October 1998 (1998-10), pages 784-789, XP002119680 JAPANESE BIOCHEMICAL SOCIETY, TOKYO., JP ISSN: 0021-924X
- DATABASE MEDLINE [Online] US NATIONAL LIBRARY OF MEDICINE (NLM), BETHESDA, MD, US YAN W ET AL: "Corin, a mosaic transmembrane serine protease encoded by a novel cDNA from human heart." retrieved from STN Database accession no. 1999262646 XP002119684 & JOURNAL OF BIOLOGICAL CHEMISTRY, (1999 MAY 21) 274 (21) 14926-35.,
- YAN W. ET AL: 'Corin, a mosaic transmembrane serine protease encoded by a novel cDNA from human heart' JOURNAL OF BIOLOGICAL CHEMISTRY vol. 274, no. 21, 21 May 1999, pages 14926 - 14935

### SEQUENCE LISTING

<110> Morse=, John
   Wu, Qingyu
   Yan, Wei
<120> NOVEL, SERINE PROTEASE NUCLEIC ACIDS AND POLYPEPTIDES
<130> SCHERING AG
<140>
   <141>
<150> 09/092,029
   <151> 1998-06-05
<160> 18
<170> PatentIn Ver. 2.0
<210> 1
   <211> 4933
   <212> DNA
   <213> Homo sapiens corin
<400> 1
   aaatcatccg tagtgcctcc ccgggggaca cgtagaggag agaaaagcga ccaagataaa 60
   agtggacaga agaataagcg agacttttta tccatgaaac agtctcctgc cctcgctccg 120
   gaagagcgct accgcagagc cgggtcccca aagccggtct tgagagctga tgacaataac 180
   atgggcaatg gctgctctca gaagctggcg actgctaacc tcctccggtt cctattgctg 240
   gtcctgattc catgtatctg tgctctcgtt ctcttgctgg tgatcctgct ttcctatgtt 300
   ggaacattac aaaaggtcta ttttaaatca aatgggagtg aacctttggt cactgatggt 360
   gaaatccaag ggtccgatgt tattcttaca aatacaattt ataaccagag cactgtggtg 420
   tctactgcac atcccgacca acacgttcca gcctggacta cggatgcttc tctcccaggg 480
   gaccaaagtc acaggaatac aagtgcctgt atgaacatca cccacagcca gtgtcagatg 540
   ctgccctacc acgccacgct gacacctctc ctctcagttg tcagaaacat ggaaatggaa 600
   aagttcctca agtttttcac atatctccat cgcctcagtt gctatcaaca tatcatgctg 660
   tttggctgta ccctcgcctt ccctgagtgc atcattgatg gcgatgacag tcatggactc 720
   ctgccctgta ggtccttctg tgaggctgca aaagaaggct gtgaatcagt cctggggatg 780
   gtgaattact cctggccgga tttcctcaga tgctcccagt ttagaaacca aactgaaagc 840
   agcaatgtca gcagaatttg cttctcacct cagcaggaaa acggaaagca attgctctgt 900
   ggaaggggtg agaactttct gtgtgccagt ggaatctgca tccccgggaa actgcaatgt 960
   aatggctaca acgactgtga cgactggagt gacgaggctc attgcaactg cagcgagaat 1020
   ctgtttcact gtcacacagg caagtgcctt aattacagcc ttgtgtgtga tggatatgat 1080
   gactgtgggg atttgagtga tgagcaaaac tgtgattgca atcccacaac agagcatcgc 1140
   tgcggggacg ggcgctgcat cgccatggag tgggtgtgtg atggtgacca cgactgtgtg 1200
   gataagtccg acgaggtcaa ctgctcctgt cacagccagg gtctggtgga atgcagaaat 1260
   ggacaatgta tccccagcac gtttcaatgt gatggtgacg aggactgcaa ggatgggagt 1320
   gatgaggaga actgcagcgt cattcagact tcatgtcaag aaggagacca aagatgcctc 1380
   tacaatccct gccttgattc atgtggtggt agctctctct gtgacccgaa caacagtctg 1440
   aataactgta gtcaatgtga accaattaca ttggaactct gcatgaattt gccctacaac 1500
   agtacaagtt atccaaatta ttttggccac aggactcaaa aggaagcatc catcagctgg 1560
   gagtcttctc ttttccctgc acttgttcaa accaactgtt ataaatacct catgttcttt 1620
   tcttgcacca ttttggtacc aaaatgtgat gtgaatacag gcgagcgtat ccctccttgc 1680
   agggcattgt gtgaacactc taaagaacgc tgtgagtctg ttcttgggat tgtgggccta 1740
   cagtggcctg aagacacaga ttgcagtcaa tttccagagg aaaattcaga caatcaaacc 1800
   tgcctgatgc ctgatgaata tgtggaagaa tgctcaccta gtcatttcaa gtgccgctca 1860
   ggacagtgtg ttctggcttc cagaagatgt gatggccagg ccgactgtga cgatgacagt 1920
   gatgaggaaa actgtggttg taaagagaga gatctttggg aatgtccatc caataaacaa 1980
   tgtttgaagc acacagtgat ctgcgatggg ttcccagact gccctgatta catggacgag 2040
   aaaaactgct cattttgcca agatgatgag ctggaatgtg caaaccatgc gtgtgtgtca 2100
   cgtgacctgt ggtgtgatgg tgaagccgac tgctcagaca gttcagatga atgggactgt 2160
   gtgaccctct ctataaatgt gaactcctct tcctttctga tggttcacag agctgccaca 2220
   gaacaccatg tgtgtgcaga tggctggcag gagatattga gtcagctggc ctgcaagcag 2280
   atgggtttag gagaaccatc tgtgaccaaa ttgatacagg aacaggagaa agagccgcgg 2340
   tggctgacat tacactccaa ctgggagagc ctcaatggga ccactttaca tgaacttcta 2400
   gtaaatgggc agtcttgtga gagcagaagt aaaatttctc ttctgtgtac taaacaagac 2460
   tgtgggcgcc gccctgctgc ccgaatgaac aaaaggatcc ttggaggtcg gacgagtcgc 2520
   cctggaaggt ggccatggca gtgttctctg cagagtgaac ccagtggaca tatctgtggc 2580
   tgtgtcctca ttgccaagaa gtgggttctg acagttgccc actgcttcga ggggagagag 2640
   aatgctgcag tttggaaagt ggtgcttggc atcaacaatc tagaccatcc atcagtgttc 2700
   atgcagacac gctttgtgaa gaccatcatc ctgcatcccc gctacagtcg agcagtggtg 2760
   gactatgaca tcagcatcgt tgagctgagt gaagacatca gtgagactgg ctacgtccgg 2820
   cctgtctgct tgcccaaccc ggagcagtgg ctagagcctg acacgtactg ctatatcaca 2880
   ggctggggcc acatgggcaa taaaatgcca tttaagctgc aagagggaga ggtccgcatt 2940
   atttctctgg aacattgtca gtcctacttt gacatgaaga ccatcaccac tcggatgata 3000
   tgtgctggct atgagtctgg cacagttgat tcatgcatgg gtgacagcgg tgggcctctt 3060
   gtttgtgaga agcctggagg acggtggaca ttatttggat taacttcatg gggctccgtc 3120
   tgcttttcca aagtcctggg gcctggcgtt tatagtaatg tgtcatattt cgtcgaatgg 3180
   attaaaagac agatttacat ccagaccttt ctcctaaact aattataagg atgatcagag 3240
   acttttgcca gctacactaa aagaaaatgg ccttcttgac tgtgaagagc tgcctgcaga 3300
   gagctgtaca gaagcacttt tcatggacag aaatgctcaa tcgtgcactg caaatttgca 3360
   tgtttgtttt ggactaattt ttttcaattt attttttcac cttcattttt ctcttatttc 3420
   aagttcaatg aaagacttta caaaagcaaa caaagcagac tttgtccttt tgccaggcct 3480
   aaccatgact gcagcacaaa attatcgact ctggcgagat ttaaaatcag gtgctacagt 3540
   aacaggttat ggaatggtct cttttatcct atcacaaaaa aagacataga tatttaggct 3600
   gattaattat ctctaccagt ttttgtttct caagctcagt gcatagtggt aaatttcagt 3660
   gttaacattg gagacttgct tttctttttc tttttttata ccccacaatt cttttttatt 3720
   acacttcgaa ttttagggta cacgagcaca acgtgcaggt tagttacata tgtatacatg 3780
   tgccatgttg gtgtgctgaa cccagtaact cgtcatttga tttattaaaa gccaagataa 3840
   tttacatgtt taaagtattt actattaccc ccttctaatg tttgcataat tctgagaact 3900
   gataaaagac agcaataaaa gaccagtgtc atccatttag gtagcaagac atattgaatg 3960
   caaagttctt tagatatcaa tattaacact tgacattatt ggacccccca ttctggatgt 4020
   atatcaagat cataatttta tagaagagtc tctatagaac tgtcctcata gctgggtttg 4080
   ttcaggatat atgagttggc tgattgagac tgcaacaact acatctatat ttatgggcaa 4140
   tattttgttt tacttatgtg gcaaagaact ggatattaaa ctttgcaaaa gagaatttag 4200
   atgagagatg caatttttta aaaagaaaat taatttgcat ccctcgttta attaaattta 4260
   tttttcagtt ttcttgcgtt catccatacc aacaaagtca taaagagcat attttagagc 4320
   acagtaagac tttgcatgga gtaaaacatt ttgtaatttt cctcaaaaga tgtttaatat 4380
   ctggtttctt ctcattggta attaaaattt tagaaatgat ttttagctct aggccacttt 4440
   acgcaactca atttctgaag caattagtgg taaaaagtat ttttccccac taaaaaactt 4500
   taaaacacaa atcttcatat atacttaatt taattagtca ggcatccatt ttgcctttta 4560
   aacaactagg attccctact aacctccacc agcaacctgg actgcctcag cattccaaat 4620
   agatactacc tgcaatttta tacatgtatt tttgtatctt ttctgtgtgt aaacatagtt 4680
   gaaattcaaa aagttgtagc aatttctata ctattcatct cctgtccttc agtttgtata 4740
   aacctaagga gagtgtgaaa tccagcaact gaattgtggt cacgattgta tgaaagttca 4800
   agaacatatg tcagttttgt tacagttgta gctacatact caatgtatca acttttagcc 4860
   tgctcaactt aggctcagtg aaatatatat attatactta ttttaaataa ttcttaatac 4920
   aaataaaatg gta                                                    4933
<210> 2
   <211> 1042
   <212> PRT
   <213> Homo sapiens corin
<400> 2
<210> 3
   <211> 3547
   <212> DNA
   <213> Murine corin
<400> 3
   gtggcagacg gtccctcact cctgtggctt ggcgtcggag acgctggcag tcatgggcag 60
   ggtttccttc agcgttcggg tcagctccgt gcggagagcc cgctgctctt gtcctgggcg 120
   atgctacctc tcctgcagag tccctccaac caccgccctc cgtgcactga acggtcttgg 180
   ctgcgcgggg gttccggggg agactgcagg tggagccgtc ggacccggcc ccttggggac 240
   ccgtggcttc ctctccgggt ccaagttcca ggctcccggc agctggaagg attgctttgg 300
   agccccgcct gctccagacg tcttgagagc agacaggagc gtgggcgagg gctgtcctca 360
   gaagctggtg actgctaact tgctgcgctt cctcctgctg gtgctcatcc cctgcatctg 420
   cgccctcatc gtgctgctgg ccatcctgct gtcctttgtg ggaacattaa aaagggttta 480
   tttcaaatca aatgacagtg aacctttggt cactgatggg gaagctcgag tgcctggtgt 540
   tattcctgta aatacagttt attatgagaa cacaggggcg ccctctctgc cccccagcca 600
   gtccactcca gcctggacac cgagagctcc ttctccagag gaccagagtc acaggaacac 660
   aagcacctgc atgaacatca ctcacagcca gtgtcaaatt ctgccctacc acagcacgtt 720
   ggcacctctc ttgccaattg tcaaaaacat ggacatggag aagttcctca agttcttcac 780
   gtacctccat cgcctcagtt gctatcaaca tatcctgctc ttcggctgta gcctcgcctt 840
   ccctgagtgc gttgttgatg gcgatgacag gcaaggtctt ctaccctgta gatctttctg 900
   tgaggctgct aaagaaggat gcgaatctgt cctgggaatg gtgaactcct cctggccgga 960
   ttccctcaga tgctctcagt ttagggacca cactgagact aacagcagtg tcagaaagag 1020
   ctgcttctca ctgcagcagg aacatggaaa gcaatcactc tgtggagggg gcgagagctt 1080
   cctgtgtacc agcgggctct gcgtccccaa gaagctgcag tgtaacggct ataatgactg 1140
   tgatgactgg agcgacgagg cgcattgcaa ctgcagcaag gatctgtttc actgtggcac 1200
   aggcaagtgc ctccattaca gcctcttgtg tgatgggtac gatgactgtg gggacctgag 1260
   tgacgagcaa aactgtgatt gtaatctcac aaaagagcat cgctgtggag atgggcgctg 1320
   cattgcggct gagtgggtgt gcgatgggga ccatgactgt gtggacaagt ctgatgaggt 1380
   caactgctct tgtcacagcc agggcctggt ggaatgcaga agtggacagt gcatccctag 1440
   caccttccag tgtgatgggg acgaagactg taaggatggg agtgacgagg agaactgcag 1500
   tgacagtcag acgccatgtc cagaaggaga acagggatgc cttggcagtt cctgcgtcga 1560
   atcctgtgct ggtagctctc tgtgtgactc agacagcagc ctgagtaact gcagtcaatg 1620
   tgagcccatc actttggaac tctgcatgaa tttgccctac aaccatacac attatccaaa 1680
   ttaccttggc cacagaactc aaaaggaagc gtccatcagc tgggagtcat ccctttcccc 1740
   tgcccttgta caaaccaact gttacaaata cctcatgttt ttcgcttgca ccattttggt 1800
   tccaaagtgt gatgtgaata caggacaacg catcccgcct tgcagactcc tgtgtgagca 1860
   ctccaaagag cgctgtgagt ctgttctggg aatcgttggc ctgcagtggc ctgaagacac 1920
   cgactgcaat caatttccag aggaaagttc agacaatcaa acttgcctcc tgcccaatga 1980
   agatgtggaa gaatgctctc cgagtcactt caaatgccgc tcgggacgat gcgttctggg 2040
   ctccaggaga tgtgacggcc aggctgactg tgacgacgac agtgacgagg agaactgtgg 2100
   ttgtaaagag agagctcttt gggaatgtcc atttaataag caatgtctga agcatacatt 2160
   aatctgcgat gggtttccag attgtccaga cagtatggat gaaaaaaact gctcattttg 2220
   ccaagacaat gagctggaat gtgccaacca tgagtgtgtg ccgcgtgacc tttggtgcga 2280
   cggatgggtc gactgctcag acagttctga tgaatggggc tgtgtgaccc tctctaaaaa 2340
   tgggaactct tcctcattgc tgactgttca caaatctgca aaggaacacc acgtgtgtgc 2400
   tgacggctgg cgggagacgt tgagtcagct ggcctgcaag cagatgggtt taggagaacc 2460
   gtctgtgacc aagctgatcc caggacagga aggccagcag tggctgaggt tgtaccccaa 2520
   ctgggagaat ctcaatggga gcaccttgca ggagctgctg gtatacaggc actcctgccc 2580
   aagcagaagt gagatttccc ttctgtgctc caagcaagac tgtggccgcc gccctgctgc 2640
   ccgaatgaac aagaggatcc ttgggggtcg gactagtcgt cctgggaggt ggccgtggca 2700
   gtgctctctg cagagtgaac ccagtggaca tatctgtggc tgtgtcctca ttgccaagaa 2760
   gtgggtcctg acagttgccc attgctttga agggagagaa gacgctgatg tttggaaagt 2820
   ggtatttggc ataaacaacc tggaccatcc atcaggcttc atgcagaccc gctttgtgaa 2880
   gaccatcctg ctacatcccc gttacagtcg agcagtggta gactatgata tcagcgtggt 2940
   ggagctgagc gatgatatca atgagacaag ctacgtcaga cctgtctgcc tacccagtcc 3000
   ggaggagtat ctagaaccag atacgtactg ctacatcaca ggctggggcc acatgggcaa 3060
   taaaatgccc tttaagctgc aggagggaga ggtccgcatt atccctctgg agcagtgcca 3120
   gtcctatttt gacatgaaga ccatcaccaa tcggatgatc tgtgctggct atgagtctgg 3180
   caccgtggac tcctgcatgg gagacagcgg tgggcctctg gtttgtgaac gacccggagg 3240
   acagtggaca ttatttggtt taacttcatg gggctccgtc tgcttttcca aagttctggg 3300
   acctggagtg tacagcaatg tgtcttactt tgtgggctgg attgaaagac aaatatatat 3360
   ccagaccttt ctccaaaaga aatcccaagg ataatcagag actttgtggg gaaacctaca 3420
   tggagaatga ccctctgaaa cagaagcttg tcctgccaag agctgtacga acaggcgttt 3480
   cacggacagg acgctcaaca tgcaccgcaa gatctctcct gtttgtgcta gatgagtttt 3540
   actcagg                                                           3547
<210> 4
   <211> 1113
   <212> PRT
   <213> Murine corin
<400> 4
   Met Gly Arg Val Ser Phe Ser Val Arg Val Ser Ser Val Arg Arg Ala
   1 5 10 15
   Arg Cys Ser Cys Pro Gly Arg Cys Tyr Leu Ser Cys Arg Val Pro Pro
   20 25 30
   Thr Thr Ala Leu Arg Ala Leu Asn Gly Leu Gly Cys Ala Gly Val Pro
   35 40 45
   Gly Glu Thr Ala Gly Gly Ala Val Gly Pro Gly Pro Leu Gly Thr Arg
   50 55 60
   Gly Phe Leu Ser Gly Ser Lys Phe Gln Ala Pro Gly Ser Trp Lys Asp
   65 70 75 80
   Cys Phe Gly Ala Pro Pro Ala Pro Asp Val Leu Arg Ala Asp Arg Ser
   85 90 95
   Val Gly Glu Gly Cys Pro Gln Lys Leu Val Thr Ala Asn Leu Leu Arg
   100 105 110
   Phe Leu Leu Leu Val Leu Ile Pro Cys Ile Cys Ala Leu Ile Val Leu
   115 120 125
   Leu Ala Ile Leu Leu Ser Phe Val Gly Thr Leu Lys Arg Val Tyr Phe
   130 135 140
   Lys Ser Asn Asp Ser Glu Pro Leu Val Thr Asp Gly Glu Ala Arg Val
   145 150 155 160
   Pro Gly Val Ile Pro Val Asn Thr Val Tyr Tyr Glu Asn Thr Gly Ala
   165 170 175
   Pro Ser Leu Pro Pro Ser Gln Ser Thr Pro Ala Trp Thr Pro Arg Ala
               180                 185                 190
   Pro Ser Pro Glu Asp Gln Ser His Arg Asn Thr Ser Thr Cys Met Asn
           195                 200                 205
   Ile Thr His Ser Gln Cys Gln Ile Leu Pro Tyr His Ser Thr Leu Ala
       210                 215                 220
   Pro Leu Leu Pro Ile Val Lys Asn Met Asp Met Glu Lys Phe Leu Lys
   225                 230                 235                 240
   Phe Phe Thr Tyr Leu His Arg Leu Ser Cys Tyr Gln His Ile Leu Leu
                   245                 250                 255
   Phe Gly Cys Ser Leu Ala Phe Pro Glu Cys Val Val Asp Gly Asp Asp
               260                 265                 270
   Arg Gln Gly Leu Leu Pro Cys Arg Ser Phe Cys Glu Ala Ala Lys Glu
           275                 280                 285
   Gly Cys Glu Ser Val Leu Gly Met Val Asn Ser Ser Trp Pro Asp Ser
       290                 295                 300
   Leu Arg Cys Ser Gln Phe Arg Asp His Thr Glu Thr Asn Ser Ser Val
   305                 310                 315                 320
   Arg Lys Ser Cys Phe Ser Leu Gln Gln Glu His Gly Lys Gln Ser Leu
                   325                 330                 335
   Cys Gly Gly Gly Glu Ser Phe Leu Cys Thr Ser Gly Leu Cys Val Pro
               340                 345                 350
   Lys Lys Leu Gln Cys Asn Gly Tyr Asn Asp Cys Asp Asp Trp Ser Asp
           355                 360                 365
   Glu Ala His Cys Asn Cys Ser Lys Asp Leu Phe His Cys Gly Thr Gly
       370                 375                 380
   Lys Cys Leu His Tyr Ser Leu Leu Cys Asp Gly Tyr Asp Asp Cys Gly
   385                 390                 395                 400
   Asp Leu Ser Asp Glu Gln Asn Cys Asp Cys Asn Leu Thr Lys Glu His
                   405                 410                 415
   Arg Cys Gly Asp Gly Arg Cys Ile Ala Ala Glu Trp Val Cys Asp Gly
               420                 425                 430
   Asp His Asp Cys Val Asp Lys Ser Asp Glu Val Asn Cys Ser Cys His
           435                 440                 445
   Ser Gln Gly Leu Val Glu Cys Arg Ser Gly Gln Cys Ile Pro Ser Thr
       450                 455                 460
   Phe Gln Cys Asp Gly Asp Glu Asp Cys Lys Asp Gly Ser Asp Glu Glu
   465                 470                 475                 480
   Asn Cys Ser Asp Ser Gln Thr Pro Cys Pro Glu Gly Glu Gln Gly Cys
                   485                 490                 495
   Leu Gly Ser Ser Cys Val Glu Ser Cys Ala Gly Ser Ser Leu Cys Asp
   500 505 510
   Ser Asp Ser Ser Leu Ser Asn Cys Ser Gln Cys Glu Pro Ile Thr Leu
   515 520 525
   Glu Leu Cys Met Asn Leu Pro Tyr Asn His Thr His Tyr Pro Asn Tyr
   530 535 540
   Leu Gly His Arg Thr Gln Lys Glu Ala Ser Ile Ser Trp Glu Ser Ser
   545 550 555 560
   Leu Phe Pro Ala Leu Val Gln Thr Asn Cys Tyr Lys Tyr Leu Met Phe
   565 570 575
   Phe Ala Cys Thr Ile Leu Val Pro Lys Cys Asp Val Asn Thr Gly Gln
   580 585 590
   Arg Ile Pro Pro Cys Arg Leu Leu Cys Glu His Ser Lys Glu Arg Cys
   595 600 605
   Glu Ser Val Leu Gly Ile Val Gly Leu Gln Trp Pro Glu Asp Thr Asp
   610 615 620
   Cys Asn Gln Phe Pro Glu Glu Ser Ser Asp Asn Gln Thr Cys Leu Leu
   625 630 635 640
   Pro Asn Glu Asp Val Glu Glu Cys Ser Pro Ser His Phe Lys Cys Arg
   645 650 655
   Ser Gly Arg Cys Val Leu Gly Ser Arg Arg Cys Asp Gly Gln Ala Asp
   660 665 670
   Cys Asp Asp Asp Ser Asp Glu Glu Asn Cys Gly Cys Lys Glu Arg Ala
   675 680 685
   Leu Trp Glu Cys Pro Phe Asn Lys Gln Cys Leu Lys His Thr Leu Ile
   690 695 700
   Cys Asp Gly Phe Pro Asp Cys Pro Asp Ser Met Asp Glu Lys Asn Cys
   705 710 715 720
   Ser Phe Cys Gln Asp Asn Glu Leu Glu Cys Ala Asn His Glu Cys Val
   725 730 735
   Pro Arg Asp Leu Trp Cys Asp Gly Trp Val Asp Cys Ser Asp Ser Ser
   740 745 750
   Asp Glu Trp Gly Cys Val Thr Leu Ser Lys Asn Gly Asn Ser Ser Ser
   755 760 765
   Leu Leu Thr Val His Lys Ser Ala Lys Glu His His Val Cys Ala Asp
   770 775 780
   Gly Trp Arg Glu Thr Leu Ser Gln Leu Ala Cys Lys Gln Met Gly Leu
   785 790 795 800
   Gly Glu Pro Ser Val Thr Lys Leu Ile Pro Gly Gln Glu Gly Gln Gln
   805 810 815
   Trp Leu Arg Leu Tyr Pro Asn Trp Glu Asn Leu Asn Gly Ser Thr Leu
   820 825 830
   Gln Glu Leu Leu Val Tyr Arg His Ser Cys Pro Ser Arg Ser Glu Ile
   835 840 845
   Ser Leu Leu Cys Ser Lys Gln Asp Cys Gly Arg Arg Pro Ala Ala Arg
   850 855 860
   Met Asn Lys Arg Ile Leu Gly Gly Arg Thr Ser Arg Pro Gly Arg Trp
   865 870 875 880
   Pro Trp Gln Cys Ser Leu Gln Ser Glu Pro Ser Gly His Ile Cys Gly
   885 890 895
   Cys Val Leu Ile Ala Lys Lys Trp Val Leu Thr Val Ala His Cys Phe
   900 905 910
   Glu Gly Arg Glu Asp Ala Asp Val Trp Lys Val Val Phe Gly Ile Asn
   915 920 925
   Asn Leu Asp His Pro Ser Gly Phe Met Gln Thr Arg Phe Val Lys Thr
   930 935 940
   Ile Leu Leu His Pro Arg Tyr Ser Arg Ala Val Val Asp Tyr Asp Ile
   945 950 955 960
   Ser Val Val Glu Leu Ser Asp Asp Ile Asn Glu Thr Ser Tyr Val Arg
   965 970 975
   Pro Val Cys Leu Pro Ser Pro Glu Glu Tyr Leu Glu Pro Asp Thr Tyr
   980 985 990
   Cys Tyr Ile Thr Gly Trp Gly His Met Gly Asn Lys Met Pro Phe Lys
   995 1000 1005
   Leu Gln Glu Gly Glu Val Arg Ile Ile Pro Leu Glu Gln Cys Gln Ser
   1010 1015 1020
   Tyr Phe Asp Met Lys Thr Ile Thr Asn Arg Met Ile Cys Ala Gly Tyr
   1025 1030 1035 1040
   Glu Ser Gly Thr Val Asp Ser Cys Met Gly Asp Ser Gly Gly Pro Leu
   1045 1050 1055
   Val Cys Glu Arg Pro Gly Gly Gln Trp Thr Leu Phe Gly Leu Thr Ser
   1060 1065 1070
   Trp Gly Ser Val Cys Phe Ser Lys Val Leu Gly Pro Gly Val Tyr Ser
   1075 1080 1085
   Asn Val Ser Tyr Phe Val Gly Trp Ile Glu Arg Gln Ile Tyr Ile Gln
   1090 1095 1100
   Thr Phe Leu Gln Lys Lys Ser Gln Gly
   1105 1110
<210> 5
   <211> 24
   <212> DNA
   <213> Human corin
<400> 5
   cagttggttt gaacaagtgc aggg 24
<210> 6
   <211> 24
   <212> DNA
   <213> Human Corin
<400> 6
   tgcaaggagg gatacgctcg cctg 24
<210> 7
   <211> 24
   <212> DNA
   <213> Human corin
<400> 7
   aatcccaaga acagactcac agcg 24
<210> 8
   <211> 24
   <212> DNA
   <213> Human corin
<400> 8
   cgggtcacag agagagctac cacc 24
<210> 9
   <211> 24
   <212> DNA
   <213> Human corin
<400> 9
   ggtctccttc ttgacatgaa tctg 24
<210> 10
   <211> 30
   <212> DNA
   <213> Human corin
<400> 10
   aacaaaacga tccttggagg tcggacgagt 30
<210> 11
   <211> 24
   <212> DNA
   <213> Mouse corin
<400> 11
   tcttctgtgt actaaacaag actg 24
<210> 12
   <211> 24
   <212> DNA
   <213> Mouse corin
<400> 12
   aggccccagg actttggaaa agca 24
<210> 13
   <211> 24
   <212> DNA
   <213> Mouse corin
<400> 13
   acagtggcct gaagacacag attg 24
<210> 14
   <211> 24
   <211> DNA
   <213> Human corin
<400> 14
   acagagcatc gctgcgggga cggg 24
<210> 15
   <211> 30
   <212> DNA
   <213> Human corin
<400> 15
   aacaaaagga tccttggagg tcggacgagt 30
<210> 16
   <211> 23
   <212> DNA
   <213> Human corin
<400> 16
   cggagcccca tgaagttaat cca 23
<210> 17
   <211> 24
   <212> DNA
   <213> Human GAPDH
<400> 17
   gtcaatgtcc caaacgtcac caga 24
<210> 18
   <211> 29
   <212> DNA
   <213> Human GAPDH
<400> 18
   atttcgggaa tgctgagaaa acagacaga 29

## Description

### BACKGROUND OF THE INVENTION

Serine proteases participate in a variety of developmental and physiological processes (Stroud R. Sci. Am. 231:74-88, 1974: Neurath H. Science 224:350-357, 1984). For instance, serine proteases are involved in cell signaling, cell differentiation, and the conversion of pro-hormones to biologically-active forms. See, e.g., Hong and Hashimoto, Cell, 82:785-794. 1995: Inagami. J. Biol. Chem., 264:3043-3046. 1989.

WO 98/03665 discloses nucleic acid and amino acid sequences of a novel human kallikrein and the use of these sequences in the diagnosis, study, prevention and treatment of disease. Tomita. Y. et al., J. Biochem., 124:784-789, 1998, identified a low-density-lipoprotein-receptor-related protein with type-II-membrane-protein-like structure in the heart of mice. WO 98/36054 discloses among others a polypeptide, referred to as ATC2, a member of the serine protease family with homology to hepsin, protasin and acrosin.

### DESCRIPTION OF THE INVENTION

The present invention relates to nucleic acids, polypeptides, and fragments thereof, of a novel gene. e.g.. a serine protease, especially a mammalian serine protease; such as human and mouse corin, which contains one or more frizzled. LDLR, scavenger receptor cysteine-rich repeats, and serine protease catalytic domains. The invention further relates to methods of using such nucleic acids and polypeptides in therapeutics, diagnostics, and research. For example, the nucleic acids and polypeptides of corin can be utilized in methods to identify modulators of its activity and in animal models to mimic human disease, and in the diagnosis of pathological conditions.

### BRIEF DESCRIPTION OF THE DRAWINGS

SEQ ID NO: 1 shows a nucleotide sequence for human corin.
SEQ ID NO: 2 shows a deduced amino acid sequence of human corin.
SEQ ID NO: 3 shows a nucleotide sequence for mouse corin.
SEQ ID NO: 4. shows a deduced amino acid sequence for mouse corin.

Fig. 1 is a schematic illustrating the arrangement of functional domains within a human corin polypeptide.
Fig. 2 shows an amino acid alignment of frizzled ("Corin Crd1" and "Corin Crd2") domains from a human corin polypeptide with Frizzled, Fz-1. and lin-17.
Fig 3 shows an amino acid sequence alignment of LDLR-repeats identified within a human corin polypeptide with a consensus sequence for human LDLR.
Fig. 4 shows an amino acid sequence alignment of a human corin serine protease domain ("Corin") with serine protease domains present in three other proteins. KAL is kalkrein. ENTK is enterokinase. TRP is trypsin.

### DETAILED DESCRIPTION OF THE INVENTION

Nucleic acid and polypeptide sequences have been identified which code for corin, a novel gene comprising a transmembrane/signal peptide, frizzled domains, low density liprotein receptor repeats (LDLR), scavenger receptor cysteine-rich repeats (SRCR), and a serine protease catalytic domain. See, e.g., Fig. 1.

In accordance with the present invention, a corin polypeptide has an immunogenic activity which is specific for corin: or, an amino acid sequence which is obtainable from a naturally-occurring source and which has one or more of the following activities or domains: a serine protease catalytic activity; a serine protease catalytic activity domain; a serine protease binding substrate activity; a pro-atrial natriuretic factor (ANF) converting enzyme catalytic activity; a frizzled domain; a LDLR repeat domain; and scavenger receptor cysteine-rich repeats (SRCR).

The polypeptide fragments coded for by the following nucleic acid fragments from the Unigene database [PubEST] are excluded: Hs.62794 (AA126468 [1686098], AA126648 [1686206], AA625395 [2537780], AA046682 [1524579]. AA249850 [1881137], and AA046793 [1524691]), and Hs.71798 (AA147031 [1716421]). (The bracketed numbers refer to the PubEST database.) The following fragments can also be excluded: Hs.121626 (AA771958), Hs.1657 (M69297), and Hs.47712 (AA203291), g1231787; g1312726; g1337948; and g942724. The nucleotide sequences of the aforementioned nucleic acids can be identified be searching publicly available databases. However, polypeptides which contain or comprise these sequences are not excluded, e.g., full-length corin, a polypeptide having two or more of these mentioned fragments, or a polypeptide having one of the mentioned fragments and additional amino acid sequences, either from corin or from another source.

By the term "immunogenic activity specific for corin, " it is meant that the corin polypeptide elicits an immunological response which is selective for corin. Such response can be cellular or humoral. Thus, the stimulation of antibodies. T-cells, macrophages, B-cells, dendritic cells, etc., by a corin amino acid sequence selected from a mammalian corin polypeptide, e.g., human corin as shown in SEQ ID NO: 2, is a specific immunogenic activity. These responses can be measured routinely. See also, discussion below on antibodies specific-for corin.

Serine protease catalytic activity means, e.g., that the corin polypeptide possesses a polypeptide cleavage activity, preferably at a peptide bond, e.g., where a serine residue of corin participates in the cleavage of the peptide bond. See, e.g., Stroud. Sci. Am., 231:74-88, 1974; Kraut, Ann. Rev. Biochem., 46:331-358, 1977. The entire corin polypeptide sequence shown in SEQ ID NO: 2 or 4, or a part of it, can possess serine protease catalytic activity. Cleavage after amino acid position 801 of SEQ ID NO: 2 (splitting the peptide bond between arginine and isoleucine) can increase or enhance such catalytic activity, resulting in the release of a catalytic fragment comprising, or consisting essentially of, amino acid positions 802-1042.

A "serine protease catalytic activity domain" refers to a region of a polypeptide which is capable of serine protease catalytic activity, as described above, but which does not necessarily possess complete, if any, activity in the background in which it is present. For instance, members of the trypsin family are often initially expressed as a pro-enzyme which exhibits complete activity only upon cleavage at a specific site within the polypeptide. Cleavage results in release of a proteolytic fragment. Generally, the "released fragment" is held in place by disulfide bonds with the remaining portion of the protein. See, SEQ ID NO: 2, e.g., for cysteines which could form the disulfide bonds. A serine protease catalytic domain refers to the proteolytic fragment prior to its release from the complete corin sequence.

A "pro-atrial natriuretic factor (ANF) converting enzyme catalytic activity" means, e.g., a catalytic activity in which pro-ANF is converted by proteolytic cleavage into one or more smaller peptides. See, e.g., Inagami, J. Biol. Chem., 264:3043-3046, 1989; Rosenzweig and Seidman, Ann. Rev. Biochem., 60:229-255, 1991; Wilkins et al., Lancet, 349:1307-1310, 1997. ANF (also, known as ANP) is a cardiac hormone that, e.g., regulates body fluid homeostasis, blood pressure, plasma volume, and other physiological processes involved in heart and kidney function. Other substrates for the converting activity, can also include homologs and proteins homologous to ANF, such as BNP and CNP. See, e.g., Wilkins et al., Lancet, 349:1307-1310, 1997; Levin et al., New Eng. J. Med., 339: 321-328. 1998. The converting activity can be measured conventionally, e.g., as described in the examples where cleavage of pro-ANF is assayed by detecting a difference in molecular weight of pro-ANF before and after treatment with corin, or with a biologically-active fragment of corin. Since corin can be expressed on the cell-surface, intact cells can be used to process substrates, such as pro-ANF.

Substrate binding is generally considered the first step in enzyme catalysis because the substrate, acting as a ligand, must first attach to the enzyme surface to enable the enzyme to carry out its catalytic reactions. This enzyme surface can be referred to as the active site of the enzyme. Binding of the substrate to the enzyme surface can involve multiple interactions with the enzyme, e.g., chemical bonding with one or more amino acids and/or functional groups which comprise the enzyme. A serine protease substrate binding activity as used herein means that a substrate, e.g., (H-D-Pro-Phe-Arg-pNA.2HC1), S2444 (pyroGlu-Gly-Arg-pNA.HCl), and S2288 (H-D-Ile-Pro-Arg-pNA.2HCl), respectively, or pro-ANF, attaches specifically to a surface of a corin polypeptide. Attachment to the enzyme can be accomplished by one or more of the interactions which hold its naturally-occurring substrate to it: however, a polypeptide can have a substrate binding activity when it holds the substrate with less than the naturally-occurring number and quality of interactions. A serine protease substrate binding activity can optionally be effective: to achieve catalysis of the substrate, to competitively or noncompetitively bind to the active site, to irreversibly attach to the enzyme, to result in the loss of catalytic activity (e.g., where it is a suicide substrate), etc.

Serine protease substrate binding activity can be measured conventionally. For instance, a competition binding assay can be employed to identify substrates which attach to a polypeptide, or derivative thereof, e.g., by combining under effective conditions, a substrate containing a detectable marker, a human corin polypeptide, or fragments thereof, and a compound which is to be tested for substrate binding activity. The assay can be accomplished in liquid phase, where bound and free substrate are separated by a membrane, or, it can be accomplished in solid phase, as desired. Solid-phase assays can be performed using high-throughput procedures, e.g., on chips, wafers, etc. Substrate binding and catalytic activity can be dissociated from each other. Thus, a corin polypeptide can possess substrate binding activity but not a catalytic activity.

A corin polypeptide can also comprise a "frizzled-like cysteine rich domain" (or "frizzled domain", as used herein) having Wnt binding activity. A frizzled domain can possess a ligand binding activity or region, e.g., as defined and described in Zorn, Current Biology, 7:R501-R504, 1997. For example, a frizzled domain can act as a receptor for ligands having homology to Wnts and other secreted glycoproteins involved in cell proliferation, cell signaling, etc. It can be membrane bound or soluble. In a soluble form, it can also act as antagonist. e.g., when the free frizzled domain of corin binds the free ligand and prevents it from interacting with its cognate receptor on the cell surface.

An LDLR repeat contains a human LDLR consensus sequence as shown in Fig. 7 and can optionally have ligand binding activity.

A corin polypeptide can also comprise a scavenger receptor cysteine-rich repeat domain ("SRCR"). See. e.g., Matsumoto et al, Proc. Natl. Acad. Sci., 87:9133-9137, 1990.

A mammalian corin is a mammalian polypeptide having an amino acid sequence which is obtainable from a natural source and the mentioned activities. It can be full-length (i.e., as shown in SEQ ID NO: 2) or it can be less than full-length and possess one or more of the mentioned activities. It therefore includes naturally-occurring normal, mutant, polymorphic, etc., sequences. Natural sources include, e.g., living cells, e.g., obtained from tissues or whole organisms, cultured cell lines, including primary and immortalized cell lines, biopsied tissues, etc.

The present invention also describes fragments of a full-length mammalian corin, such as human or mouse corin. The fragments are preferably "biologically active". By "biologically active", it is meant that the polypeptide fragment possesses an activity in a living system or with components of a living system. Biological activities include those mentioned, e.g., a catalytic activity, a substrate binding activity, and/or an immunogenic activity. Fragments can be prepared according to any desired method, including, chemical synthesis, genetic engineering, cleavage products, etc. See, below.

The present invention also relates to a human corin having a deduced sequence of amino acids 1 to 1042 amino acids as shown in SEQ ID NO: 2. The 1042 amino acid polypeptide has a predicted molecular weight of about 116 kilodaltons. It comprises the following domains: hydrophobic region at about amino acid positions 46-66; frizzled cysteine-rich domains at about amino acid positions 134-259 and 450-573; seven LDLR repeats at about amino acid positions 268-415 and 579-690; a cysteine-rich region at about amino acid positions 713-801 homologous to a macrophage scavenger receptor motif; and a serine protease catalytic domain at about amino acid positions 802-1042. There are nineteen putative N-glycosylation sites present in the extracellular domain which is at about amino acid positions 67-1042.

A corin polypeptide of the invention, e.g., having an amino acid sequence as shown in SEQ ID NO: 2, can by analyzed by available methods to identify other structural and/or functional domains in the polypeptide. For example, a corin polypeptide can be analyzed by methods disclosed in. e.g., Kyte and Doolittle, J. Mol. Bio.,157:105, 1982; EMBL Protein Predict; Rost and Sander. Proteins, 19:55-72. 1994.

The hydrophobic domains at about amino acids 46-66 of a human corin can serve as a membrane anchoring sequence. See, also, hepsin, a mammalian serine protease of the trypsin family which contains a transmembrane domain near its amino-terminus (Kurachi et al., Methods in Enzymology 244:100-114, 1994); Stubble-stubbloid, a *Drosophila melanogaster* serine protease which also contains a transmembrane domain (Appel et al.. Proc. Natl. Acad. Sci. USA 90:4937-4941, 1993). There are positively charged amino acid residues immediately preceding the hydrophobic and putative transmembrane domain, indicating that corin may be a type 11 transmembrane protein with the amino terminus in the cytosol.

Corin also contains at least two cysteine-rich frizzled domains at about amino acid positions 134-259 and 450-573. Fig. 2 shows a comparison of the human corin frizzled domains with FZ-1. lin-17, and Frizzled. The frizzled domain comprises, e.g., a ligand binding site. See, e.g., Zorn, Current Biology, 7:R501-R504, 1997; Leyns et al.. Cell, 88:747-756, 1997. As described below, an aspect of the invention is to identify ligands which bind to the corin-frizzled domains, and which optionally regulate the activity of cells expressing the corin polypeptide or cells expressing the ligand.
Analysis of the corin protein sequence showed that in the extracellular region there are two frizzled-like cysteine-rich domains, seven LDL receptor repeats, one macrophage scavenger receptor-like domain, and one trypsin-like serine protease domain (2A). Two frizzled-like cysteine-rich domains are located at amino acids 134-259 and 450-573, respectively. Amino acid sequences of these two domains share significant similarities with the extracellular cysteine-rich domain of the Drosophila Frizzled protein, a seven transmembrane receptor essential for polarity determination during the development of the fruitfly (Vinson et al., Nature, 338:263-264, 1989). The frizzled-like cysteine-rich domains have also been found in other proteins, such as Dfz2 in Drosophila (Bhanot et al., Nature, 382:225-230;1996), Lin-17 in C. elegans (Sawa et al., Genes Dev., 10:2189-2197, 1996) and FZ-1 in human (Chan et al., J. Biol. Chem., 267:25202-25207, 1992). The sequences of the two frizzled-like cysteine-rich domains in corin are closest to those in Lin-17 and FZ-1. All the ten conserved cysteine residues are present in the frizzled-like cysteine-rich domains of corin. Between amino acids 268-415 and 579-690, there are seven cysteine-rich repeats homologous to the LDL receptor class A repeats (Brown et al., Nature, 388:629-630,1997). Each repeat is about 36 amino acids long and contains six cysteine residues as well as a highly conserved cluster of negatively charged amino acids. In the LDL receptor, these cysteine-rich repeats bind calcium ions and play an essential role in endocytosis of the extracellular ligands (*ibid*.). Similar motifs have been found in the extracellular domain of other membrane receptors, such as LDL receptor-related protein (LRP1) (Krieger and Herz, Annu. Rev. Biochem., 63:601-637, 1994), megalin (also known as LRP2 or gp330)(Kounnas et al., J. Biol. Chem., 268:14176-14181, 1993), complement proteins (Catterall et al., Biochem. J., 242:849-856, 1987), enterokinase (Kitamoto et al., Proc. Natl. Acad. Sci. U.S.A., 91:7588-7592, 1994), and Drosophila proteins yolkless and nudel (Schonbaum et al., Proc. Natl. Acad. Sci. U.S.A., 92:1485-1489; 1995; Hong and Hashimoto, Cell, 82:785-794, 1995). In addition to the frizzled-like cysteine-rich domains and LDL receptor-like repeats, there is another cysteine-rich region between amino acids 713 and 801 in corin. This region contains 88 amino acids and is homologous to the cysteine-rich motif found in the macrophage scavenger receptor (Matsumoto et al., Proc. Natl. Acad. Sci. U S.A., 87:9133-9137, 1990). This motif is also present in the sea urchin spermatozoa speract receptor (Thorpe and Garbers, J. Biol. Chem., 264:6545-6549, 1989; Dangott et al., Proc. Natl. Acad. Sci. U.S.A., 86:2128-2132, 1989) and the vertebrate serine protease, enterokinase (Kitamoto et al., Proc. Natl. Acad. Sci. U.S.A., 91:7588-7592, 1994). At the carboxyl terminus of corin protein between amino acid residues 802 and 1042, there is a trypsin-like serine protease domain. This protease domain is highly homologous to the catalytic domain of members of the trypsin superfamily. For example, amino acid sequence identities between corin and prekallikrein (Chung et al., Biochemistry, 25:2410-2417,1986), factor XI (Fujikawa et al., Biochemistry, 25:2417-2424, 1986) and hepsin (Leytus et al., Biochemistry, 27:1067-1074, 1988) are 40%, 40% and 38%, respectively. All essential features of serine protease sequences are well conserved in corin. The active site residues of the catalytic triad are located at His843, Asp892 and Ser985. The amino acid residues forming the substrate specificity pocket are located at Asp979, Gly1007 and Gly1018. These residues are predicted to bind the substrate P1 residues, suggesting that corin would cleave its substrate after basic residues, such as lysine or arginine. In addition, a putative activation cleavage site was found at Arg801, suggesting that corin would be synthesized as an inactive zymogen and that another trypsin-like enzyme was required for its activation.
In the protease domain, there are 12 cysteine residues. Potential pairing of these cysteine residues can be predicted by comparing with other well-studied serine proteases, such as trypsin and chymotrypsin. First three pairs of cysteine residues present in essentially all members of the trypsin superfamily are located at Cys828-Cys844, Cys955-Cys970 and Cys981-Cys1010. Two more pairs of cysteine residues are present at the positions Cys790-Cys912 and Cys926-Cys991. These two pairs of cysteine residues are commonly found in a subfamily of two-chain serine proteases, such as chymotrypsin and prekallikrein (Chung et al., Biochemistry, 25:2410-2417, 1986). The presence of Cys790 and Cys912 indicated that, after the activation cleavage at Arg801, the catalytic domain of corin would remain attached to the rest of molecule by a disulfide bond. Interestingly, there is one additional pair of cysteine residues, Cys817 and Cys830, present in corin. Cysteine residues at these two positions were not found in any other serine proteases in vertebrates. A search of databases showed that a chymotrypsinogen-like serine protease from the lugworm, Arenicola marina, had two cysteine residues at the corresponding positions (Kyte and Doolittle, J. Mol. Biol., 157:105-132, 1982). A model of the corin protease domain was built based on the structure of bovine chymotrypsinogen A. Based on this corin model, where the Cα atoms of these two cysteine residues were held fixed during energy minimization, the distance between the sulfur atoms of their side chains is about 2.5 A after rotamer searching. The model indicates that these two cysteines are likely to form a disulfide bond connecting two b-sheets in the core of the protease domain.

In addition to the human corin sequence, a corin from another mammalian species, mouse, has been cloned and identified. A full-length nucleotide and amino acid sequence of mouse corin is shown in SEQ ID NOS: 3 and 4. There are 1113 amino acids in the mouse corin and it comprises the same domains as human corin. There is 89% amino acid sequence identity between mouse and human corin.

Other corin homologs from mammalian and non-mammalian sources can be obtained according to various methods. For example, hybridization with an oligonucleotide selective for a mammalian corin can be employed to select such homologs, e.g., as described in Sambrook et al., Molecular Cloning, 1989, Chapter 11. Such homologs can have varying amounts of nucleotide and amino acid sequence identity and similarity to corin. Non-mammalian organisms include, e.g., vertebrates, invertebrates, zebra fish, chicken. *Drosophila, C. elegans, Xenopus, S. pombe, S. cerevisiae,* roundworms, prokaryotes, plants, *Arabidopsis,* viruses, etc.

The invention also describes corin specific amino acid sequences, e.g., a defined amino acid sequence which is found in the particular human sequence of SEQ ID NO: 2, but not in other amino acid sequences from non-corin polypeptides. A specific amino acid sequence can be found routinely, e.g., by searching a gene/protein database using the BLAST set of computer programs. A corin specific amino acid sequence can be useful to produce peptides as antigens to generate an immune response specific for it. Antibodies obtained by such immunization can be used as a specific probe for a mammalian corin protein for diagnostic or research purposes.

As mentioned, polypeptides of the present invention comprise a complete human coding sequence for a corin. Useful fragments include, e.g., fragments comprising, or consisting essentially, of the frizzled domain, one or more LDLR domains, SRCR repeats the transmembrane domain, the serine catalytic domain or fragment. A preferred fragment of human corin comprises the polypeptide sequence of SEQ ID NO: 2, with the proviso that the fragment is not a polypeptide coded for by Hs.62794 (AA126468 [1686098], AA126648 [1686206], AA625395 [2537780], AA046682 [1524579], AA249850 [1881137], and AA046793 [1524691]), and Hs.71798 (AA147031 [1716421)). See, above.

A fragment of a corin polypeptide can be selected to have a specific biological activity, e.g., a serine protease activity, a pro-ANF converting enzyme activity, a substrate binding activity, an immunogenic activity, etc. A useful fragment can be identified routinely by testing such fragments for a desired activity. The measurement of these activities is described below and in the examples. These peptides can also be identified and prepared as described in EP 496 162.

A polypeptide of the present invention can also have 100% or less amino acid sequence identity to the amino acid sequence set forth in SEQ ID NO:2. For the purposes of the following discussion: Sequence identity means that the same nucleotide or amino acid which is found in the sequence set forth in SEQ ID NOS: 1-2 is found at the corresponding position of the compared sequence(s). A polypeptide having less than 100% sequence identity to the amino acid sequence set forth in SEQ ID NO: 2 can contain various substitutions from the naturally-occurring sequence, including homologous and non-homologous amino acid substitutions. See below for examples of homologous amino acid substitution. The sum of the identical and homologous residues divided by the total number of residues in the sequence over which the corin polypeptide is compared is equal to the percent sequence similarity. For purposes of calculating sequence identity and similarity, the compared sequences can be aligned and calculated according to any desired method, algorithm, computer program, etc., including, e.g., FASTA, BLASTA. A polypeptide having less than 100% amino acid sequence identity to the amino acid sequence of SEQ ID NO: 2 can comprise e.g., about 99%, 98%, 97%, 95%, 90%, 70% etc. A preferred amount of amino acid sequence identity is about 85% or more, e.g., about 86%.

A mammalian corins polypeptide, fragment, or substituted polypeptide can also comprise various modifications, where such modifications include lipid modification, methylation, phosphorylation, glycosylation, covalent modifications (e.g., of an R-group of an amino acid), amino acid substitution, amino acid deletion, or amino acid addition. Modifications to the polypeptide can be accomplished according to various methods, including recombinant, synthetic, chemical, etc.

Polypeptides of the present invention (e.g.. human corin or mouse corin, fragments thereof, mutations thereof) can be used in various ways, e.g., in assays, as immunogens for antibodies as described below, as biologically-active agents (e.g., having one or more of the activities associated with corin).

A polypeptide coding for a corin, a derivative thereof, or a fragment thereof, can be combined with one or more structural domains, functional domains, detectable domains, antigenic domains, and/or a desired polypeptide of interest, in an arrangement which does not occur in nature, i.e., not naturally-occurring, e.g., as in a human or murine corin gene, a genomic fragment prepared from the genome of a living organism, e.g., an animal, preferably a mammal, such as human, mouse, or cell lines thereof. A polypeptide comprising such features is a chimeric or fusion polypeptide. Such a chimeric polypeptide can be prepared according to various methods, including, chemical, synthetic, quasi-synthetic, and/or recombinant methods. A chimeric nucleic acid coding for a chimeric polypeptide can contain the various domains or desired polypeptides in a continuous (e.g., with multiple N-terminal domains to stabilize or enhance activity) or interrupted open reading frame, e.g., containing introns, splice sites, enhancers, etc. The chimeric nucleic acid can be produced according to various methods. See, e.g., U.S. Pat. No. 5,439,819, A domain or desired polypeptide can possess any desired property, including, a biological function such as catalytic, signalling, growth promoting, cellular targeting (e.g., signal sequence, targeting sequence, such as to endosomes, lysosomes. ER. nucleus), etc., a structural function such as hydrophobic, hydrophilic, membrane-spanning, etc., receptor-ligand functions, and/or detectable functions, e.g., combined with enzyme, fluorescent polypeptide, green fluorescent protein, (Chalfie et al., 1994, Science, 263:802; Cheng et al., 1996, Nature Biotechnology, 14:606; Levy et al., 1996, Nature Biotechnology, 14:610, etc. In addition, a polypeptide, or a part of it, can be used as a selectable marker when introduced into a host cell. For example, a nucleic acid coding for an amino acid sequence according to the present invention can be fused in frame to a desired coding sequence and act as a tag for purification, selection, or marking purposes. The region of fusion can encode a cleavage site to facilitate expression, isolation, purification, etc.

A polypeptide according to the present invention can be produced in an expression system, e.g., in vivo, in vitro, cell-free, recombinant, cell fusion, etc., according to the present invention. Modifications to the polypeptide imparted by such systems include glycosylation, amino acid substitution (e.g., by differing codon usage), polypeptide processing such as digestion, cleavage, endopeptidase or exopeptidase activity, attachment of chemical moieties, including lipids and phosphates, etc.

A polypeptide according to the present invention can be recovered from natural sources, transformed host cells (culture medium or cells) according to the usual methods, including, detergent extraction (e.g., Triton-X-100 CHAPS, octylglucoside), ammonium sulfate or ethanol precipitation, acid extraction, anion or cation exchange chromatography, phosphocellulose chromatography, hydrophobic interaction chromatography, hydroxyapatite chromatography and lectin chromatography. Protein refolding steps can be used, as necessary, in completing the configuration of the mature protein. Finally, high performance liquid chromatography (HPLC) can be employed for purification steps. A corin polypeptide can also be isolated as described for other serine proteases, e.g., Wu et al., J. Biol. Chem., 267: 24408-24412, 1992; Wu et al., Proc. Natl. Acad. Sci., 88:6775-6779, 1991.

A mammalian corin nucleic acid, or fragment thereof, is a nucleic acid having a nucleotide sequence obtainable from a natural source. See, above. It therefore includes naturally-occurring, normal, mutant, polymorphic alleles, degenerate sequences, etc. Natural sources include, e.g., living cells obtained from tissues and whole organisms, cultured cell lines, including primary and immortalized cell lines. Preferably, the following nucleic acid fragments from the Unigene database [PubEST] are excluded: Hs.62794 (AA 126468 [1686098], AA 126648 [1686206], AA625395 [2537780], AA046682 [1524579], AA249850 [1881137], and AA046793 [1524691]), and Hs.71798 (AA 147031 [1716421]). (The bracketed numbers refer to the PubEST database.) Optionally, the following fragments can also be excluded: Hs.121626 (AA771958), Hs.1657 (M69297), and Hs.47712 (AA203291), g1231787; g1312726; g1337948; and g942724. The nucleotide sequences of the aforementioned nucleic acids can be identified be searching publicly available databases. However, nucleic acids which contain or comprise these sequences are not excluded, e.g., full-length corin, or, a nucleic acid having two or more of these mentioned fragments. It is also preferred that the mentioned fragments in a cloning vector, such as a plasmid or phage, are excluded.

Human corin is expressed as an about a 5 kb mRNA. It is most abundant in heart. It is either absent, or expressed in very low levels in brain, placenta, lung, liver, skeletal muscle, kidney, pancreas, spleen, thymus, prostate, testis, ovary, small intestine, colon, bladder, uterus, and stomach. Consequently, corin can be used as a marker for the presence of heart tissue, e.g., in tissue sections (using corin-specific antibodies or corin-specific nucleic acid probes), in biopsied samples, etc. Corin was also detected in various human cell lines, including uterus tumor, osteosarcoma, endometrium carcinoma lines HEC-1-A, AN3 CA, and RL95-2, leiomyosarcoma SK-LM-1, and osteosarcoma U2-OS. Further details are described in the examples below.

A nucleic acid sequence of a human corin allele having 4933 base pairs is shown in SEQ ID NO: 1. The size of the DNA is consistent with the length of corin mRNA (-5 kb) detected by Northern analysis. An ATG codon is located at position 95 that may represent the translation initiation site. The open reading frame (ORF) spans 3126 bp with a 5' untranslated region (UTR) of 94 nucleotides before the initiation codon. At the 3' end, there is a 1.7-kb 3' UTR after the stop codon at position 3221. A polyadenylylation signal of AATAAA is present 12 nucleotides before the poly (A)⁺tail. A nucleic acid sequence of the invention can contain the complete coding sequence from amino acid 1 to amino acid 1042, degenerate sequences thereof, and fragments thereof. A nucleic acid according to the present invention can also comprise a nucleotide sequence which is 100% complementary, e.g., an anti-sense, to any nucleotide sequence mentioned above and below.

The present invention also describes a mouse nucleotide sequence coding for all or part of a corin, e.g., as shown in SEQ ID NO: 3. As is the case for the human allele, the invention relates to degenerate sequences thereof, and anti-sense fragments thereof. Northern analysis shows a prominent transcript at about 5 kb in samples derived from the heart. In contrast to Northen analysis with human samples, low levels of mRNA were detected in samples obtained from mouse testes and kidneys. In situ hybridization with mouse corin mRNA was also detected in embryonic heart tissue, atrium and ventricular myocytes, developing kidneys, and cartilage-derived structures. Further details are described in the examples.

A nucleic acid according to the present invention can be obtained from a variety of different sources. It can be obtained from DNA or RNA. such as polyadenylated mRNA, e.g.. isolated from tissues, cells, or whole organism. The nucleic acid can be obtained directly from DNA or RNA, or from a cDNA library. The nucleic acid can be obtained from a cell at a particular stage of development, having a desired genotype, phenotype (e.g., an embryonic or adult heart cell or tissue), etc.

As described for corin polypeptides mentioned above, a nucleic acid comprising a nucleotide sequence coding for a polypeptide according to the present invention can include only coding sequence; a coding sequence and additional coding sequence (e.g., sequences coding for leader, secretory, targeting, enzymatic, fluorescent or other diagnostic peptides), coding sequences and non-coding sequences, e.g., untranslated sequences at either a 5' or 3' end, or dispersed in the coding sequence, e.g., introns. A nucleic acid comprising a nucleotide sequence coding without interruption for a polypeptide means that the nucleotide sequence contains an amino acid coding sequence for a corin, with no non-coding nucleotides interrupting or intervening in the coding sequence, e.g., absent intron(s). Such a nucleotide sequence can also be described as contiguous. A genomic DNA coding for a mammalian human or mouse corin, etc., can be obtained routinely.

A nucleic acid according to the present invention also can comprise an expression control sequence operably linked to a nucleic acid as described above. The phrase "expression control sequence" means a nucleic acid sequence which regulates expression of a polypeptide coded for by a nucleic acid to which it is operably linked. Expression can be regulated at the level of the mRNA or polypeptide. Thus, the expression control sequence includes mRNA-related elements and protein-related elements. Such elements include promoters, enhancers (viral or cellular), ribosome binding sequences, transcriptional terminators, etc. An expression control sequence is operably linked to a nucleotide coding sequence when the expression control sequence is positioned in such a manner to effect or achieve expression of the coding sequence. For example, when a promoter is operably linked 5' to a coding sequence, expression of the coding sequence is driven by the promoter. Expression control sequences can be heterologous or endogenous to the normal gene.

A nucleic acid in accordance with the present invention can be selected on the basis of nucleic acid hybridization. The ability of two single-stranded nucleic acid preparations to hybridize together is a measure of their nucleotide sequence complementarity, e.g., base-pairing between nucleotides, such as A-T, G-C, etc. The invention thus also relates to nucleic acids which hybridize to a nucleic acid comprising a nucleotide sequence as set forth in SEQ ID NO: 1. A nucleotide sequence hybridizing to the latter sequence will have a complementary nucleic acid strand, or act as a template for one in the presence of a polymerase (i.e.. an appropriate nucleic acid synthesizing enzyme). The present invention includes both strands of nucleic acid, e.g., a sense strand and an anti-sense strand.

Hybridization conditions can be chosen to select nucleic acids which have a desired amount of nucleotide complementarity with the nucleotide sequence set forth in SEQ ID NO: 1. A nucleic acid capable of hybridizing to such sequence, preferably, possesses, e.g., about 85%, more preferably, 90%, 92%, and even more preferably, 95%, 97%, or 100% complementarity, between the sequences. The present invention particularly relates to nucleic acid sequences which hybridize to the nucleotide sequence set forth in SEQ ID NO: 1 under low or high stringency conditions.

Nucleic acids which hybridize to corin sequences can be selected in various ways. For instance, blots (i.e., matrices containing nucleic acid) can be incubated in a prehybridization solution (6X SSC, 0.5% SDS, 100 ug/ml denatured salmon sperm DNA. 5X Denhardt's solution, and 50% formamide), at 30EC. overnight, and then hybridized with radiolabeled probes (corin) in a hybridrization solution (6X SSC, 0.5% SDS, 100 ug/ml denatured salmon sperm DNA and 50% formamide), at 42EC overnight in accordance with known procedures. Blots can be washed at high stringency conditions that allow, e.g., for less than 5% bp mismatch (e.g., wash twice in 0.1 % SSC and 0.1% SDS for 30 min at 65EC), i.e., 95% or greater sequence identity. Whereas high stringency washes can allow for less than 5% mismatch, relaxed or low stringency wash conditions (e.g., wash twice in 0.2% SSC and 0.5% SDS for 30 min at 37EC) can permit up to 20% mismatch. Another non-limiting example of low stringency conditions includes a final wash at 42EC in a buffer containing 30 mM NaCI and 0.5% SDS. Another non-limiting example of high stringency conditions includes a final wash at 65EC in aqueous buffer containing 30 mM NaCl and 0.5% SDS. Washing and hybridization can also be performed as described in Sambrook et al., Molecular Cloning, 1989, Chapter 9. Hybridization can also be based on a calculation of melting temperature (Tm) of the hybrid formed between the probe and its target, as described in Sambrook et al. Such stringent conditions can select sequences which have, e.g., at least about 95%, preferably 97%, nucleotide complementarity between the nucleic acids, with the proviso that such nucleic acid is not: Hs.62794 (AA126468 [1686098], AA126648 [1686206], AA625395 [2537780], AA046682 [1524579], AA249850 [1881137], and AA046793 [1524691]), and Hs.71798 (AA147031 [1716421]). See, also above and below. Nucleic acids which contain or comprise these sequences are not excluded, e.g., full-length corin, or a nucleic acid having two or more of these mentioned fragments.

According to the present invention, a nucleic acid or polypeptide can comprise one or more differences in the nucleotide or amino acid sequence set forth in SEQ ID NOS: 1-2. Changes or modifications to the nucleotide and/or amino acid sequence can be accomplished by any method available, including directed or random mutagenesis.

A nucleic acid coding for a human or mouse corin according to the invention can comprise nucleotides which occur in a naturally-occurring corin gene e.g., naturally-occurring polymorphisms, normal or mutant alleles (nucleotide or amino acid), mutations which are discovered in a natural population of mammals, such as humans, monkeys, pigs, mice, rats, or rabbits. By the term naturally-occurring, it is meant that the nucleic acid is obtainable from a natural source, e.g., animal tissue and cells, body fluids, tissue culture cells, forensic samples. Naturally-occurring mutations can include deletions (e.g., a truncated amino- or carboxy-terminus), substitutions, or additions of nucleotide sequence. These genes can be detected and isolated by nucleic acid hybridization according to methods which one skilled in the art would know. A nucleotide sequence coding for a human corin polypeptide of the invention can contain codons found in a naturally-occurring gene, transcript, or cDNA, for example, e.g., as set forth in SEQ ID NO: 1, or it can contain degenerate codons coding for the same amino acid sequences. For instance, it may be desirable to change the codons in the sequence to optimize the sequence for expression in a desired host.

The present invention also relates to corin polypeptide muteins, i.e., any polypeptide which has an amino acid sequence which differs in amino acid sequence from an amino acid sequence obtainable from a natural source (a fragment of a mammal corin does not differ in amino acid sequence from a naturaily-occurring conn). Thus, corin muteins comprise amino acid substitutions, insertions, and deletions, including non-naturally occurring amino acids. See, e.g., Wu et al., Proc. Natl. Acad. Sci., 88:7775-6779, 1991, especially reporting amino acid substitutions where a basic amino acid was substituted with glutamic acid, for guidance on making mutations.

Muteins to a corin amino acid sequence of the invention can also be prepared based on homology searching from gene data banks, e.g., Genbank, EMBL. Sequence homology searching can be accomplished using various methods, including algorithms described in the BLAST family of computer programs, the Smith-Waterman algorithm, etc.

A mutein(s) can be introduced into a sequence by identifying and aligning amino acids within a domain which are identical and/or homologous between polypeptides and then modifying an amino acid based on such alignment. For instance, sequence comparisons between human corin and the Frizzled, LDLR, and serine protease domains of other proteins are illustrated in Figs 2-4. These alignments reveal amino acid positions which are identical and also amino acid positions where the residues differ from each other but are homologous. Homologous amino acids can be defined based on the size of the side chain and degree of polarization, including, small nonpolar: cysteine, proline, alanine, threonine; small polar: serine, glycine, aspartate, asparagine: large polar: glutamate, glutamine, lysine, arginine; intermediate polarity: tyrosine, histidine, tryptophan; large nonpolar: phenylalanine, methionine, leucine, isoleucine, valine.

Homologous acids can also be grouped as follows: uncharged polar R groups, glycine, serine, threonine, cysteine, tyrosine, asparagine, glutamine; acidic amino acids (negatively charged), aspartic acid and glutamic acid; basic amino acids (positively charged), lysine, arginine, histidine. Homologous amino acids also include those described by Dayhoff in the Atlas of Protein Sequence and Structure 5 (1978), and by Argos in EMBO J., 8, 779-785 (1989).

Muteins in accordance with the present invention include amino acid sequences where a residue in the human or mouse corin sequence is replaced by a homologous residue from a corresponding domain.

Thus, the present invention relates to a corin nucleotide sequence of SEQ ID NO: 2, wherein said nucleic acid codes for a polypeptide and one or more amino acid positions are substituted or deleted, or both, and the polypeptide coded for by the nucleic acid has a serine protease catalytic domain activity or an immunogenic activity specific for corin. Such nucleic acid can contain one or more substituted amino acid positions which are substituted by homologous amino acids.

In addition, the sequence alignments as illustrated in Figs. 2-4 also provide information on amino acid substitutions that would be expected to reduce, decrease, or, eliminate a biological activity. For instance, where alignment reveals identical amino acids conserved between two or more domains (e.g.. replacing the conserved H, D, or S residues indicated in Fig. 4), elimination or substitution of the amino acid(s) would affect its biological activity. Mutations in the catalytic triad, namely, His910. Asp959, and Ser1052 (mouse corin), abolish the catalytic activity of serine proteases.

A corin polypeptide mutein, and its corresponding nucleotide coding sequence, can have an amino acid sequence as set forth in SEQ ID NO: 2, except where one or more positions are substituted by homologous amino acids, e.g., where there are 1, 5, 10, 15, or 20 substitutions. The invention also relates to mutein polypeptides and mutein nucleic acids coding for such polypeptides.

A nucleic acid according to the present invention can comprise. e.g., DNA. RNA, synthetic nucleic acid, peptide nucleic acid, modified nucleotides, or mixtures. A DNA can be double- or single-stranded. Nucleotides comprising a nucleic acid can be joined via various known linkages, e.g., ester, sulfamate, sulfamide, phosphorothioate, phosphoramidate, methylphosphonate, carbamate, etc., depending on the desired purpose, e.g., resistance to nucleases, such as RNase H. improved *in vivo* stability, etc. See, e.g., U.S. Pat. No. 5,378,825.

Various modifications can be made to the nucleic acids, such as attaching detectable markers (avidin, biotin, radioactive elements), moieties which improve hybridization, detection, or stability. The nucleic acids can also be attached to solid supports, e.g., nitrocellulose, magnetic or paramagnetic microspheres (e.g., as described in U.S. Pat. No. 5,41 1.863; U.S. Pat. No. 5,543,289; for instance, comprising ferromagnetic, supermagnetic, paramagnetic, superparamagnetic, iron oxide and polysaccharide), nylon, agarose, diazotized cellulose, latex solid microspheres, polvacrylamides, etc., according to a desired method. See, e.g., U.S. Pat. Nos. 5,470,967; 5,476,925; 5,478.893.

Another aspect relates to oligonucleotides and nucleic acid probes. Such oligonucleotides or nucleic acid probes can be used, e.g., to detect, quantitate, or isolate a mammalian corin nucleic acid in a test sample. The nucleic acids can be utilized as oligonucleotide probes, e.g., in PCR, in RACE, differential display, in combination with cDNA libraries, expression libraries, etc. Useful oligonucleotides are described below in the examples. Detection can be desirable for a variety of different purposes, including research, diagnostic, and forensic. For diagnostic purposes, it may be desirable to identify the presence or quantity of a such a nucleic acid sequence in a sample, where the sample is obtained from tissue, cells, body fluids, etc. The present invention describes a method of detecting a nucleic acid comprising, contacting a target nucleic acid in a test sample with an oligonucleotide under conditions effective to achieve hybridization between the target and oligonucleotide; and detecting hybridization. An oligonucleotide as described in the invention can also be used in synthetic nucleic acid amplification such as PCR (e.g., Saiki et al., 1988. Science, 241:53; U.S. Pat. No. 4,683,202; PCR Protocols: A Guide to Methods and Applications, Innis et a]., eds., Academic Press, New York, 1990) or differential display (See, e.g., Liang et al., Nucl. Acid. Res., 21:3269-3275, 1993; U.S. Pat. No. 5,599,672; WO97/18454) or RACE. Such detection can be accomplished in combination with oligonucleotides for other genes, e.g., genes involved in cardiac tissue or bone development or function.

Another aspect of the present invention is a nucleotide sequence which is unique to human corin. By a unique sequence to a corin, it is meant a defined order of nucleotides which occurs in corin, e.g., in the nucleotide sequence of SEQ ID NO: 1, but rarely or infrequently in other nucleic acids, especially not in an animal nucleic acid, preferably mammal, such as human, rat, mouse, etc. Both sense and antisense nucleotide sequences are included. A unique nucleic acid according to the present invention can be determined routinely. A nucleic acid comprising such a unique sequence can be used as a hybridization probe to identify the presence of, e.g., human or mouse corin, in a sample comprising a mixture of nucleic acids, e.g., on a Northern blot. Hybridization can be performed under stringent conditions (see, above) to select nucleic acids having at least 95% identity (i.e., complementarity) to the probe, but less stringent conditions can also be used. A unique corin nucleotide sequence can also be fused in-frame, at either its 5' or 3' end, to various nucleotide sequences as mentioned throughout the patent, including coding sequences for other parts of corin, enzymes, GFP, etc, expression control sequences, etc.

Hybridization can be performed under different conditions, depending on the desired selectivity, e.g., as described in Sambrook et al., Molecular Cloning, 1989. For example, to specifically detect human or mouse corin, an oligonucleotide can be hybridized to a target nucleic acid under conditions in which the oligonucleotide only hybridizes to it, e.g., where the oligonucleotide is 100% complementary to the target. Different conditions can be used if it is desired to select target nucleic acids which have less than 100% nucleotide complementarity, at least about, e.g., 99%. 97%, 95%, 90%, 70%, 67%.

Oligonucleotides are described which can comprise any continuous nucleotide sequence of SEQ ID NO: 1. These oligonucleotides (nucleic acid) according to the present invention can be of any desired size, e.g., about 10-200 nucleotides, 12-100, preferably 12-50, 12-25, 14-16, at least about 15, at least about 20, etc. The oligonucleotides can have non-naturally-occurring nucleotides, e.g., inosine. The oligonucleotides can have 100% identity or complementarity to a sequence of SEQ ID NO: 1, or it can have mismatches or nucleotide substitutions, e.g., 1. 2, 3, 4, or 5 substitutions. In accordance with the present invention, the oligonucleotide can comprise a kit, where the kit includes a desired buffer (e.g., phosphate, tris, etc.), detection compositions, etc. The oligonucleotide can be labeled or unlabeled, with radioactive or non-radioactive labels as known in the art.

Anti-sense nucleic acid can also be prepared from a nucleic acid according to the present invention, preferably an anti-sense to a coding sequence of SEQ ID NO: 1. Antisense nucleic acid can be used in various ways, such as to regulate or modulate expression of corin, e.g., inhibit it, to detect its expression, or for *in situ* hybridization. These oligonucleotides can be used analogously to U.S. Pat. No. 5,576,208. For the purposes of regulating or modulating expression of corin, an anti-sense oligonucleotide can be operably linked to an expression control sequence.

The nucleic acid according to the present invention can be labeled according to any desired method. The nucleic acid can be labeled using radioactive tracers such as ³²P, ³⁵S, ¹²⁵I, ³H, or ¹⁴C, to mention some commonly used tracers. The radioactive labeling can be carried out according to any method such as, for example, terminal labeling at the 3' or 5' end using a radiolabeled nucleotide, polynucleotide kinase (with or without dephosphorylation with a phosphatase) or a ligase (depending on the end to be labeled). A non-radioactive labeling can also be used, combining a nucleic acid of the present invention with residues having immunological properties (antigens, haptens), a specific affinity for certain reagents (ligands), properties enabling detectable enzyme reactions to be completed (enzymes or coenzymes, enzyme substrates, or other substances involved in an enzymatic reaction), or characteristic physical properties, such as fluorescence or the emission or absorption of light at a desired wavelength, etc.

A nucleic acid according to the present invention, can be used to detect expression of corin in whole organs, tissues, cells, etc., by various techniques, including Northern blot, PCR, RACE, *in situ* hybridization, etc. Such nucleic acids can be particularly useful to detect disturbed expression, e.g., cell-specific and/or subcellular alterations, of corin. The levels of corin can be determined alone or in combination with other gene products, especially cardiac specific gene products.

A nucleic acid according to the present invention can be expressed in a variety of different systems, *in vitro* and *in vivo,* according to the desired purpose. For example, a nucleic acid can be inserted into an expression vector, introduced into a desired host, and cultured under conditions effective to achieve expression of a polypeptide coded for by the nucleic acid. Effective conditions include any culture conditions which are suitable for achieving production of the polypeptide by the host cell, including effective temperatures, pH, medias, additives to the media in which the host cell is cultured (e.g., additives which amplify or induce expression such as butyrate, or methotrexate if the coding nucleic acid is adjacent to a dhfr gene), cycloheximide, cell densities, culture dishes, etc. A nucleic acid can be introduced into the cell by any effective method including, e.g., naked DNA. calcium phosphate precipitation, electroporation, injection. DEAE-Dextran mediated transfection, fusion with liposomes, association with agents which enhance its uptake into cells, viral transfection. A cell into which a nucleic acid of the present invention has been introduced is a transformed host cell. The nucleic acid can be extrachromosomal or integrated into a chromosome(s) of the host cell. It can be stable or transient. An expression vector is selected for its compatibility with the host cell. Host cells include, mammalian cells, e.g., COS-7. CV1, BHK, CHO, HeLa. LTK. NIH 3T3, yeast, insect cells, such as Sf9 (S. frugipeda) and Drosophila, bacteria, such as E. coli, Streptococcus, bacillus, yeast, fungal cells, plant cells, embryonic stem cells (e.g., mammalian, such as mouse or human), bone cells (such as, osteoclasts or chondrocytes), cardiac cells (e.g., from a primary culture), muscle cells, neuronal cells, etc. Expression control sequences are similarly selected for host compatibility and a desired purpose, e.g., high copy number, high amounts, induction, amplification, controlled expression. Other sequences which can be employed include enhancers such as from SV40, CMV, RSV, inducible promoters, cell-type specific elements, or sequences which allow selective or specific cell expression. Promoters that can be used to drive its expression, include, e.g., the endogenous promoter. MMTV, SV40; trp, lac, tac, or T7 promoters for bacterial hosts: or alpha factor, alcohol oxidase, or PGH promoters for yeast.

Another gene of interest can be introduced into the same host for purposes of, e.g., modulating corin function. Such genes can be the normal gene, or a variation, e.g., a mutation, chimera, polymorphism, etc.

A nucleic acid or polypeptide of the present invention can be used as a size marker in nucleic acid or protein electrophoresis, chromatography, etc. Defined restriction fragments can be determined by scanning the sequence for restriction sites, calculating the size, and performing the corresponding restriction digest. A corin cDNA as shown in SEQ ID NO: 1 can be used as a 4.8 kb molecular weight marker in nucleic acid electrophoresis.

The human corin cDNA as shown in SEQ ID NO: 1 maps to human chromosome position 4p12-13. It can thus be used as a marker in pedigree mapping. For example, this chromosomal region appears to segregate with total anomalous pulmonary venous return (TAPVR) and therefore could be used to map the disease in association with other genetics markers. See. e.g.. Bleyl et al., Am. J. Hum. Genet., 56: 408-415, 1995.

Another aspect of the present invention relates to the regulation of biological pathways in which a corin gene, or gene product, is involved, particularly pathological and developmental conditions, and to the diagnosis of such conditions by detecting either corin polypeptide or nucleic acid. For example. ANF is involved in a variety of physiological processes related to the cardiovascular and kidney systems, including, e.g., body fluid homeostasis, blood pressure, vasodilation, natriuresis, inhibition of sodium absorption in the glomerular duct, increased glomerular filtration, inhibition of aldosterone production and secretion, mitogenesis, etc. The regulation of the pro-ANF converting activity of corin can therefore have profound effects on an organism=s physiology. For instance, ventricular levels of ANF are normally low in ventricular myocytes but increase dramatically with cardiovascular diseases. In patients with congestive heart failure, plasma levels of ANF are high. The high concentrations are correlated with the severity of ventricular dysfunction. Similarly, high concentrations of ANF are associated with cardiac arrhythmias and hemodynamic compromise. See, e.g., Levin et al., New Engl. J. Med., 339:321-328, 1998. Abnormally high levels can be reduced by inhibiting the activity of corin, e.g.. transcriptionally by inhibiting gene expression or by administering enzyme inhibitors, which act directly on catalytic activity.

Since corin is highly expressed in cartilage-derived and cardiac cells, it may be involved in diseases associated with these tissues, such as familial hypertrophic cardiomyopathy, osteopetrosis, and osteoporosis-pseudoglioma syndrome, osteoporosis, Paget=s disease, osteitis deformans, and total anomalous pulmonary venous return. As mentioned, corin oligonucleotides can be utilized in pedigree mapping to study the familial inheritance of these diseases, in analogy to TAPVR, and for diagnostic purposes (e.g., prenatal) if corin is associated (gene linkage or causative) with such a disease. Corin may also be involved heart development, developmental pathways which involve signaling with Wnt proteins and other growth factors, cell differentiation (e.g., chondrocyte differentiation), and related processes. In addition, corin may be involved in cell-cell signaling, differentiation (bone and/or cardiac), and other developmental pathways. This role may be mediated by serine protease catalytic activity, frizzled domains, and/or the LDRL domains. Corin may also be involved in the processing (e.g., by catalytic cleavage of a progrowth factor) of growth factors, such as BMP or TGF-β.

Along these lines, the invention can be used for the treatment of cardiovascular and kidney disease, such as hypertension, congestive heart disease, or renal failure (as well as pathological conditions related to any to other pathway in which corin participates), comprising the administration, to a host in need thereof, of an effective amount of an agent which modulates the activity of a mammalian corin. By the term Amodulates.≅ it is meant: increasing, agonizing, promoting, stabilizing, decreasing, reducing, antagonizing, blocking, inhibiting, etc. The nature of the desired modulatory effect can be determined routinely, e.g., on whether the pathological effect is produced by elevated or diminished quantities of ANF. The activity can be inhibited by administering an agent which directly blocks the catalytic activity of corin (e.g., an enzyme inhibitor, such as leupeptin or diisopropyl fluorophosphate) or by an agent, such as antisense, which blocks transcription or translation of the corresponding gene. Corin activity can be increased, e.g., by administering a corin gene which is effective to produce corin polypeptide. Corin gene can be administered in analogy to other gene therapies. See, e.g., Magovern et al., Hum. Gen. Ther., 8:215-227, 1997; Springer et al., Mol. Cell., 2:549-558, 1998.

The present invention also relates to methods of modulating the activity of corin *in vitro,* either by directly modulating its activity, or by modulating expression of the gene which encodes it. Enzyme activity can be measured conventionally, e.g., as described in Wu et al., J. Biol. Chem., 267:24408-24412, 1992; Wu et al., Proc. Natl. Acad. Sci., 88:6775-6779, 1991. Converting activity can be measured as described in the examples, or, e.g., as described by Inagami, J. Biol. Chem., 264:3043-3046, 1989. The invention thus relates to a method of identifying modulators of the aforementioned activity of a corin polypeptide, fragment, or mutein thereof, comprising: reacting, in the presence of a test compound, a corin polypeptide and a substrate for serine protease or the converting enzyme, under conditions effective for said polypeptide to cleave said substrate; detecting said cleavage; and identifying whether the test compound modulates said serine protease activity by comparing the amount of cleavage in the presence and absence of the test compound.

Any substrate is suitable, and, any means for detection, such as a chromogenic, fluorogenic, radioactive, electrophoretic, etc. can be utilized. For example, the products of cleavage can be detected using labeled substrates in combination with gel electrophoresis. In a preferred example, the substrate is a chromogenic substrate, i.e., a peptide that reacts with the serine protease and which is designed to possess a selectivity similar to that of the natural substrate for the enzyme. Attached to the peptide part of the chromogenic substrate is a chemical group which when released after the enzyme cleavage gives rise to a detectable color. The color change can be followed spectrophotometrically and is proportional to the proteolytic activity of the serine protease catalytic domain. These substrates can be routinely synthesized. Hydrolysis of substrate (e.g., containing 4-nitroaniline, pNA. as the chromophore) can be measured at room temperature by following absorbance at 405 nm. Substrates which can be used to measure amidolytic activity of serine proteases include, S2302 (H-D-Pro-Phe-Arg-pNA.2HCl), S2444 (pyroGlu-Gly-Arg-pNA.HCl), and S2288 (H-D-Ile-Pro-Arg-pNA.2HCl), respectively. Other chromophores that can be incorporated into the substrate include, e.g., ANBA, ADMP, thiolester derivatives, etc. See, e.g., Chromogenix catalogs and its websites for further information. Values for Kₘ and k_{cat} can be calculated by the Michaelis-Menton equation.

The invention also relates to a method of identifying compounds which bind specifically to a corin polypeptide, comprising: contacting a corin polypeptide with a test compound under conditions effective for said test compound to bind specifically to said corin polypeptide; and detecting binding to said corin polypeptide. Binding assays can be performed conventionally. The corin polypeptide comprises the complete coding sequence.

The corin component can be added to the reaction mixture in a variety forms, e.g., substantially purified, as a component of a cell, as a soluble extract, or as a lysate. In each case, the corin polypeptide can be obtained from a natural source, a recombinant source (i.e., a "recombinant" polypeptide is a produced by genetic engineering, e.g., introduced into a cell line on a plasmid, vector, naked DNA, etc., and expressed in the cell), or it can be produced synthetically (produced chemically or enzymatically. The corin polypeptide can be expressed in a mammalian cell, an insect cell line, or in bacteria, e.g., as a fusion or non-fusion protein.

Preferably, corin is expressed in a cell line transformed with a corin coding sequence (e.g., a cDNA. a gene, a genomic fragment, etc.). In the latter case, the corin is present as a heterologous component of the cell; by heterologous, it is meant that the corin is coded for by a coding sequence that has been introduced by the hand of a person into the cell, e.g., by transfection, transformation, etc. Preferably, the corin is expressed at high levels in the cell (bacterial, yeast, insect, mammalian, etc.). A human corin is a preferred coding sequence. See, e.g., SEQ ID NOS: 1 and 2.

In a preferred aspect of the invention, the corin is provided as a cell lysate, e.g., cells transformed with human corin are lysed and the resulting lysate is used directly in the assay, i.e.. a crude lysate. The crude lysate comprising the recombinant human corin can optionally be refined or enriched for human corin. For instance, membrane fractions can be isolated conventionally.

Corin can also be modulated at earlier steps in its expression pathway, e.g., modulating its transcription, mRNA stability, translation, post-translational modifications, processing (such as cleavage at the internal cleavage site), etc. Expression can be regulated using different agents, e.g., an antisense nucleic acid, a ribozyme, an aptamer, a synthetic compound, or a naturally-occurring compound.

Compounds identified in this or other manners can be useful to modulate corin activity in a cell, a tissue, a whole organism, *in situ, in vitro* (test tube, a solid support, etc.), *in vivo,* or in any desired environment. In general, a compound having such an *in vitro* activity will be useful *in vivo* to modulate a biological pathway associated with corin, e.g., to treat a pathological condition associated with the biological and cellular activities mentioned above, including bone and cardiac diseases and developmental disorders thereof, growth factor and protein maturation via serine protease activity, etc.

To treat a disease, the compound, or mixture, can be formulated into a pharmaceutical composition comprising a phamaceutically acceptable carrier and other excipients as apparent to the skilled worker. See, e.g., Remington's Pharmaceutical Sciences, Eighteenth Edition. Mack Publishing Company, 1990. Such composition can additionally contain effective amounts of other compounds.

The present invention also relates to a method of modulating, preferably inhibiting, expression of a gene coding for a mammalian corin, comprising: contacting a cell expressing a mammalian corin of the present invention, such as a human corin, with an amount of agent, such as an antisense oligonucleotide or antisense RNA of a human corin gene, which is effective to sequence-specifically inhibit said gene. Inhibiting expression of a corin gene can inhibit the maturation of ANF, and have consequent impact on the pathways in which ANF is involved, including those mentioned above.

Sequence-specific inhibition of a gene can be accomplished conventionally using antisense nucleic acid, such as antisense oligonucleotides or RNA. For example, antisense oligonucleotides, such as phosphodiester or phosphorothioate deoxyoligonucleotides can be designed to specific regions of a corin RNA, such as to the translation initiation site, and can then be administered to cells expressing such genes in quantities effective to inhibit their expression. Generally, an antisense nucleic acid is a nucleic acid which is complementary to the sense or coding strand of a given gene, and as a result are also complementary and thus able to specifically hybridize with mRNA transcripts of the gene.

To enhance stability, the administered nucleic acid can be modified, e.g., to make it resistant to cellular enzymes, oxidation, reduction, nucleases, etc, or to enhance its uptake into cells. Any suitable modification can be used, including, e.g., phosporothioates, methylphosphonates, phosphodiester oligonucleotide linked to an acridine intercalating agent and/or a hydrophobic tail, psoralen derivatives, 2'-ribose modifications, pentose sugar derivatives, nitrogen base derivatives, etc. See, e.g., U.S. Pat. No. 5,576,208 and U.S. Pat. No. 5,744.362. See, above, for other derivatives, modifications, etc. which can be useful in the invention. In general, an antisense nucleic acid of the present invention can comprise monomers of naturally-occurring nucleotides, non-naturally-occurring nucleotides, and combinations thereof to enhance cellular uptake and/or stability.

Antisense can be administered as naked nucleic acid, complexed or encapsulated with and by other agents which facilitate its uptake into a cell, injected into cells, or any suitable delivery means in association with vectors, such as viral or adeno. Antisense gene therapy can be accomplished by any suitable method. See, e.g., Phillips, Am. J. Cardiol, 82:605-625, 1998; Haller et al., Kidney Int., 53:1550-1558, 1998.

The present invention also relates to antibodies which specifically recognize full length corin. An antibody specific for corin means that the antibody recognizes a defined sequence of amino acids-within or including a corin, e.g., the human sequence of SEQ ID NO: 2. Thus, a specific antibody will generally bind with higher affinity to an amino acid sequence, i.e., an epitope, found in SEQ ID NO: 2 than to a different epitope(s), e.g., as detected and/or measured by an immunoblot assay or other conventional immunoassay. Thus, an antibody which is specific for an epitope of human corin is useful to detect the presence of the epitope in a sample, e.g., a sample of tissue containing human corin gene product, distinguishing it from samples in which the epitope is absent. Such antibodies are useful as described in Santa Cruz Biotechnology, Inc.. Research Product Catalog, and can be formulated accordingly, e.g., 100 µg/ml.

Antibodies, e.g., polyclonal, monoclonal, recombinant, chimeric, humanized, can be prepared according to any desired method. See, also, screening recombinant immunoglobulin libraries (Orlandi et al., Proc. Natl. Acad. Sci., 86: 3833-3837, 1989; Huse et al., Science, 256: 1275-1281, 1989); *in vitro* stimulation of lymphocyte populations; Winter and Milstein, Nature, 349: 293-299, 1991. For example, for the production of monoclonal antibodies, a polypeptide according to Fig. 2 or Fig. 3 can be administered to mice, goats, or rabbit subcutaneously and/or intraperitoneally, with or without adjuvant, in an amount effective to elicit an immune response. The antibodies can also be single chain or FAb fragments. The antibodies can be IgG, subtypes, IgG2a, IgG1. etc. Antibodies, and immune responses, can also be generated by administering naked DNA See, e.g., U.S. Pat. Nos. 5,703,055; 5,589,466; 5,580,859.

Corin, or fragments thereof, for use in the induction of antibodies do not need to have biological activity; however, they must have immunogenic activity. Peptides for use in the induction of corin-specific antibodies may have an amino sequence consisting of at least five amino acids, preferably at least 10 amino acids. Short stretches of corin amino acids, e.g., five amino acids, can be fused with those of another protein such as keyhole limpet hemocyanin, or another useful carrier, and the chimeric molecule used for antibody production.

Several different approaches, as mentioned, can be utilized to prepare antibodies specific for corin. For instance, in one approach, denatured corin from purified corin (e.g., purified by reverse-phase HPLC separation) is obtained in quantities up to 75 mg. This denatured protein can be used to immunize mice or rabbits using standard protocols; about 100 micrograms are adequate for immunization of a mouse, while up to 1 mg might be used to immunize a rabbit. For identifying mouse hybridomas, the denatured protein can be radioiodinated and used to screen potential murine B-cell hybridomas for those which produce antibody. This procedure requires only small quantities of protein, such that 20 mg would be sufficient for labeling and screening of several thousand clones.

In another approach, an amino acid sequence of corin, as deduced from the cDNA. is analyzed to determine regions of high immunogenicity. Polypeptides comprising these regions are synthesized and used in suitable immunization protocols to raise antibodies. Analysis to select appropriate epitopes is described by Ausubel FM et al (1989, Current Protocols in Molecular Biology, Vol 2. John Wiley & Sons). The optimal amino acid sequences for immunization are usually at the C-terminus, the N-terminus and those intervening, hydrophilic regions of the polypeptide which are likely to be exposed to the external environment when the protein is in its natural conformation. Typically, selected peptides, about 15 residues in length, are synthesized using an Applied Biosystems Peptide Synthesizer Model 431 A using fmoc-chemistry and coupled to keyhole limpet hemocyanin (KLH, Sigma) by reaction with M- maleimidobenzoyl-N-hydroxysuccinimide ester (MBS; cf. Ausubel FM et al, supra). If necessary, a cysteine may be introduced at the N-terminus of the peptide to permit coupling to KLH. Rabbits are immunized with the peptide-KLH complex in complete Freund's adjuvant. The resulting antisera are tested for antipeptide activity by binding the peptide to plastic, blocking with 1% BSA. reacting with antisera, washing and reacting with labeled (radioactive or fluorescent), affinity purified, specific goat anti-rabbit IgG..

Antibodies can also be generated against any desired region, or subregion thereof, e.g, about, or comprising, amino acids 46-66; 134-259; 450-573; 268-415; 579-690; 713-801; 802-1402.

Hybridomas can also be prepared and screened using standard techniques. Hybridomas of interest are detected by screening with labeled corin to identify those fusions producing the monoclonal antibody with the desired specificity. In a typical protocol, wells of plates (FAST, Becton-Dickinson. Palo Alto, Calif.) are coated with affinity purified, specific rabbit-anti-mouse (or suitable anti-species Ig) antibodies at 10 mg/ml. The coated wells are blocked with 1% BSA. washed and exposed to supernatants from hybridomas. After incubation the wells are exposed to labeled corin, 1 mg/ml. Clones producing antibodies will bind a quantity of labeled corin which is detectable above background. Such clones are expanded and subjected to 2 cycles of cloning at limiting dilution (1 cell/3 wells). Cloned hybridomas are injected into pristine mice to produce ascites, and monoclonal antibody is purified from mouse ascitic fluid by affinity chromatography on Protein A. Monoclonal antibodies with affinities of at least 10⁸ M. preferably 10⁹ to 10¹⁰, or stronger, will typically be made by standard procedures as described in Harlow and Lane (1988) Antibodies: A Laboratory Manual. Cold Spring Harbor Laboratory, or Goding (1986) Monoclonal Antibodies: Principles and Practice. 2nd Ed. Academic Press N.Y.

Particular corin antibodies are useful for the diagnosis of prepathologic conditions, and chronic or acute diseases which are characterized by differences in the amount or distribution of corin. Diagnostic tests for corin include methods utilizing the antibody and a label to detect corin in human (or mouse, etc, if using mouse, etc.) body fluids, tissues or extracts of such tissues (such as in heart and cartilage tissues).

The polypeptides and antibodies of the present invention may be used with or without modification. Frequently, the polypeptides and antibodies will be labeled by joining them, either covalently or noncovalently, with a substance which provides for a detectable signal. A wide variety of labels and conjugation techniques are known and have been reported extensively in both the scientific and patent literature. Suitable labels include radionuclides, enzymes, substrates, cofactors, inhibitors, fluorescent agents, chemiluminescent agents, magnetic particles and the like. Patents teaching the use of such labels include U.S. Pat. Nos. 3,817,837; 3.850.752; 3,939,350; 3,996,345; 4,277,437; 4,275,149; and 4,366,241. Also, recombinant immunoglobulins maybe produced as shown in U.S. Pat. No. 4,816,567, incorporated herein by reference.

A variety of protocols for measuring soluble or membrane-bound corin, using either polyclonal or monoclonal antibodies specific for corin are known in the art. Examples include enzyme-linked immunosorbent assay (ELISA), radioimmunoassay (RIA) and fluorescent activated cell sorting (FACS). A two-site monoclonal-based immunoassay utilizing monoclonal antibodies reactive to two non-interfering epitopes on EC is preferred, but a competitive binding assay may be employed. These assays are described, among other places, in Maddox, Del. et al (1983) J Exp Med 158: 1211.

Antibodies and other ligands which bind corin can be used in various ways, including as therapeutic, diagnostic, and commercial research tools, e.g. to quantitate the levels of corin polypeptide in animals, tissues, cells, etc., to identify the cellular localization and/or distribution of it, to purify it, or a polypeptide comprising a part of it, to modulate the function of it, in Western blots, ELISA. immunoprecipitation, RIA, etc. The present invention relates to such assays, compositions and kits for performing them, etc. Utilizing these and other methods, an antibody according to the present invention can be used to detect corin polypeptide or fragments thereof in various samples, including tissue, cells, body fluid, blood, urine, cerebrospinal fluid. A method of the present invention comprises: a) contacting a ligand which binds to a peptide of SEQ ID NO: 2 under conditions effective, as known in the art, to achieve binding, and b) detecting specific binding between the ligand and peptide. By specific binding, it is meant that the ligand attaches to a defined sequence of amino acids, e.g., within or including the amino acid sequence of SEQ ID NO: 2 or derivatives thereof.

Native or recombinant corin can be purified by immunoaffinity chromatography using corin-specific antibodies. In general, an immunoaffinity column is constructed by covalently coupling the anti-corin antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified Ig is covalently attached to a chromatographic resin such as CnBr activated Sepharose (Pharmacia LKB Biotechnology, Piscataway, N.J.). The antibody is coupled to the resin, the resin is blocked, and the derivative resin is washed according to the manufacturer's instructions.

An immunoaffinity column is utilized in the purification of corin by preparing a fraction from cells containing corin. This preparation can be derived by solubilization of the whole cell or of a subcellular fraction obtained via differential centrifugation by the addition of detergent or by other methods well known in the art. Alternatively, soluble corin containing a signal sequence may be secreted in useful quantity into the medium in which the cells are grown.

A soluble corin-containing preparation is passed over the immunoaffinity column, and the column is washed under conditions, e.g., high ionic strength buffers in the presence of detergent, that allow the preferential absorbance of corin. Then, the column is eluted under conditions that disrupt antibody/corin binding (e.g., a buffer of pH 2-3 or a high concentration of a chaotrope such as urea or thiocyanate ion), and the corin is collected.

In addition, ligands which bind to a corin polypeptide according to the present invention, or a derivative thereof, can also be prepared, e.g., using synthetic peptide libraries or aptamers (e.g., Pitrung et al., U.S. Pat. No. 5,143,854; Geysen et al., 1987. J. Immunol. Methods, 102:259-274; Scott et al., 1990, Science, 249:386; Blackwell et al., 1990. Science, 250:1104; Tuerk et al., 1990, Science, 249: 505.)

The antibodies or derivatives thereof can also be used to inhibit expression of corin or a fragment thereof. The levels of corin polypeptide can be determined alone or in combination with other gene products. In particular, the amount (e.g., its expression level) of corin polypeptide can be compared (e.g., as a ratio) to the amounts of other polypeptides in the same or different sample, e.g., actin. In general, reagents which are specific for corin can be used in diagnostic and/or forensic studies according to any desired method. e.g., as U.S. Pat. Nos. 5,397,712; 5,434,050; 5,429,947.

The present invention also relates to a corin polypeptide, prepared according to a desired method, e.g., as disclosed in U.S. Pat. No. 5,434,050. A labeled polypeptide can be used, e.g., in binding assays, such as to identify substances that bind or attach to corin, to track the movement of corin in a cell, in an *in vitro, in vivo,* or *in situ* system, etc.

A nucleic acid, polypeptide, antibody, corin, ligand etc., according to the present invention can be isolated. The term "isolated" means that the material is in a form in which it is not found in its original environment, e.g., more concentrated, more purified, separated from component, etc. An isolated nucleic acid includes, e.g., a nucleic acid having the sequence of corin separated from the chromosomal DNA found in a living animal. This nucleic acid can be part of a vector or inserted into a chromosome (by specific gene-targeting or by random integration at a position other than its normal position) and still be isolated in that it is not in a form which it is found in its natural environment. A nucleic acid or polypeptide of the present invention can also be substantially purified. By substantially purified, it is meant that nucleic acid or polypeptide is separated and is essentially free from other nucleic acids or polypeptides, i.e., the nucleic acid or polypeptide is the primary and active constituent.

The present invention also describes a non-human transgenic animal, e.g., a non-human-mammal, such as a mouse, comprising a corin or a corin knock-out. Transgenic animals can be prepared according to known methods, including, e.g., by pronuclear injection of recombinant genes into pronuclei of 1=cell embryos, incorporating an artificial yeast chromosome into embryonic stem cells, gene targeting methods, embryonic stem cell methodology. See, e.g., U.S. Patent Nos. 4,736,866; 4,873,191; 4,873,316; 5,082,779; 5,304,489; 5,174,986; 5,175.384; 5,175,385; 5,221.778; Gordon et al., Proc. Natl. Acad. Sci., 77:7380-7384 (1980); Palmiter et al., Cell, 41:343-345 (1985); Palmiter et al., Ann. Rev. Genet., 20:465-499 (1986); Askew et al., Mol. Cell. Bio., 13:4115-4124, 1993; Games et al. Nature, 373:523-527, 1995; Valancius and Smithies, Mol. Cell. Bio., 11:1402-1408, 1991; Stacey et al., Mol. Cell. Bio., 14:1009-1016, 1994; Hasty et al., Nature, 350:243-246, 1995; Rubinstein et al., Nucl. Acid Res., 21:2613-2617,1993. A nucleic acid according to the present invention can be introduced into any non-human mammal, including a mouse (Hogan et al., 1986, in Manipulating the Mouse Embryo: A Laboratory Manual, Cold Spring Harbor Laboratory. Cold Spring Harbor, New York), pig (Hammer et al., Nature. 315:343-345, 1985), sheep (Hammer et al., Nature, 315:343-345, 1985), cattle, rat, or primate. See also. e.g., Church. 1987. Trends in Biotech. 5:13-19*;* Clark et al., 1987. Trends in Biotech. 5:20-24. and DePamphilis et al., 1988. BioTechniques, 6:662-680. In addition, e.g., custom transgenic rat and mouse production is commercially available. These transgenic animals are useful animals models to test for corin function, as food for a snake, as genetic markers to detect strain origin, etc. Such transgenic animals can further comprise other transgenes or knock-outs thereof (e.g., in other serine proteases, natriuretic peptides, etc.).

Transgenic animals comprising multiple copies of the corin gene, the corin gene driven by strong promoters, or corin knockouts, can be useful as animal models for hypertension, renal disease, and cardiac disease. See, e.g., Steinhelper et al., Hypertension, 16:301-307. 1990; John et al., Am. J. Physiol., 271:R109-R114, 1996.

The present invention thus describes a transgenic animal, such as a rodent, a mouse, or a rat, comprising cells which contain a recombinant corin gene integrated into a chromosome of said cell at the native corin gene locus, said recombinant corin gene comprising a nucleotide coding sequence which codes for a recombinant corin polypeptide comprising at least one amino acid whose identity and/or position is not naturally-occurring in said native corin gene. Such a recombinant gene can be produced by homologous recombination, e.g., between a human corin and the corin gene of the host animal at its native locus.

The present invention also describes a transgenic animal, such as a mouse or a rat, comprising cells which contain at least one functionally disrupted recombinant corin gene at a chromosomal corin gene locus, wherein said disruption prevents functional expression of the corin polypeptide coded for by the corin gene. Functional inactivation or disruption refers, e.g., to a partial or complete reduction of the expression of at least a portion of a polypeptide encoded by an endogenous serine protease gene of a single cell, selected cells, or all of the cells of a mammal. Such reduction can result in concomitant reduction in pro-ANF-converting enzyme activity, causing a decrease in physiologically-active ANF. The term "knockout" is a synonym for functional inactivation of the gene.

A gene targeting strategy is described that facilitates the introduction of a desired nucleotide sequence into a corin gene. The gene targeting strategy preferably utilizes double reciprocal recombination and a positive selectable marker to assist in the insertion of the nucleotide sequence into a target nucleic acid. The target nucleic acid is preferably a gene, more preferably a gene at its particular chromosomal locus. The desired nucleotide sequence is inserted into the gene in such a way that the gene is functionally disrupted, i.e., its expression is partially or completely reduced.

Modification of the corin gene atis chromosomal locus can be accomplished according to anysuitable method, such as homologous recombination. The latter can be used to disrupt the corin gene, introduce a gene mutation into the corin gene, insert or delete DNA. etc. Selection and use of sequences effective for homologous recombination is described, e.g., in Deng and Capecchi, Mol. Cell. Bio., 12:3365-3371, 1992; Bollag et al., Annu. Rev. Genet., 23:199-225. 1989; Waldman and Liskay, Mol. Cell. Bio., 8:5350-5357, 1988: Rubinstein et al., Nucl. Acid Res., 21:2613-2617, 1993; WO94/23049; WO95/14377.

The corin gene can be completely functionally disrupted, e.g., by deleting 5' regions of the gene. Alternatively, specific regions of corin can be modified. For instance, the catalytic domain at about amino acid positions 802-1042 in the human corin gene, or corresponding positions in the mouse gene, can be genetically altered by recombination to produce a corin with modified activity, including null activity, increased activity, reduced activity.

Generally, the nucleic acids, polypeptides, antibodies, etc. of the present invention can be prepared and used as described in, U.S. Pat. Nos. 5,501.969, 5,506.133, 5.441,870; WO 90/00607; WO 91/15582.

For other aspects of the nucleic acids, polypeptides, antibodies, etc., reference is made to standard textbooks of molecular biology, protein science, and immunology. See, e.g., Davis et al. (1986), Basic Methods in Molecular Biology, Elsevir Sciences Publishing, Inc., New York; Hames et al. (1985), Nucleic Acid Hybridization, IL Press, Molecular Cloning, Sambrook et al.; Current Protocols in Molecular Biology, Edited by F.M. Ausubel et al., John Wiley & Sons, Inc: Current Protocols in Human Genetics, Edited by Nicholas C. Dracopoli et al., John Wiley & Sons, Inc.; Current Protocols in Protein Science; Edited by John E. Coligan et al., John Wiley & Sons, Inc.; Current Protocols in Immunology; Edited by John E. Coligan et al., John Wiley & Sons, Inc.

### EXAMPLES

### lsolation of human corin cDNA clones:

A partial EST sequence was identified based on analysis of Incyte EST database for novel serine protease cDNAs. The clone (307474) was ordered from Incyte. A 2.1 kb EcoRl-Xhol fragment from the clone was used to screen a human heart cDNA library (Clontech). Phagemid 14b2, which contains a 3.8 kb insert, was obtained from one positive phage clone by *in vivo* exision. Oligo primers which derived from phagemid 14b2 were used to further clone 5=-end cDNA sequence by 5' RACE, using Marathon-ready human heart cDNA (Clontech) as templates. The PCR products were cloned into pCRII vector (Invitrogen) and sequenced. The full-length (i.e., having an initiation codon and a termination codon) human corin cDNA sequence was obtained by compiling sequences obtained from 5'-RACE and phagemid using GCG DNA sequence analysis package.

### Oligo primers used in 5' RACE

PrWY109: 5'-CAGTTGGTTTGAACAAGTGCAGGG-3'
PrWY 110: 5'-TGCAAGGAGGGATACGCTCGCCTG-3'
PRWY111: 5'-AATCCCAAGAACAGACTCACAGCG-3'
PRWY118: 5'-CGGGTCACAGAGAGAGCTACCACC-3'
PRWY119: 5'-GGTCTCCTTCTTGACATGAATCTG-3'
   5'-AACAAAACGATCCTTGGAGGTCGGACGAGT-3'

### Northern Analysis:

The 2.1 kb-EcoRI-XhoI fragment of Incyte clone 307474 was labeled by ³²P-dCTP using a random primer labeling kit (Boehringer). Human Multiple Tissue Northern Blot I, Human Multiple Tissue Northern Blot II. Human Muscle Northern Blot filters (Clontech) were hybridized with the labeled human corin cDNA probe. The Northern hybridization was carried out overnight at 42EC with 40% formamide, 5X Denhardts solution, 6 X SSC, 100 µg/ml salmon sperm DNA, 0.1 % SDS. The filters were washed with 0.2 X SSC, 0.1% SDS at 60EC, and exposed to Fuji imaging plates.

### Cloning of Mouse Corin cDNA:

Mouse corin cDNA clones were isolated by a PCR-based strategy. A mouse heart cDNA library was purchased from Clontech and used as templates for PCR amplification (30 cycles of 1.5-min annealing at 55EC, 1.5-min extension at 72EC, and 1-min denaturation at 94EC). The sequences of PCR primers are based on the human corin cDNA sequences. Primers used for amplification of the mouse cDNA include:
Cor09: 5'-TCTTCTGTGTACTAAACAAGACTG-3'
Cor12: 5'-AGGCCCCAGGACTTTGGAAAAGCA-3'
Cor02: 5'-ACAGTGGCCTGAAGACACAGATTG-3'
Prwy128: 5'-ACAGAGCATCGCTGCGGGGACGGG-3'
DNA fragments from the PCR reactions were cloned into the pCRII vector. Plasmid clones that were derived from independent PCR reactions were used for further sequencing. Mouse and human corin cDNAs share over 85% sequence identities.

### In situ Hybridization:

RT-PCR-mRNA samples were isolated from Hec-1-A. U2-OS, SK-LMS-1, and AN3-CA cells using a commercial RNA preparation kit (Oligotex Direct mRNA Mini Kits, QIAGEN). First strand cDNAs were synthesized using SuperScript II RNase- reverse transcriptase (Life Technologies). Human corin specific oligonucleotide primers (sense primer: 5'-AACAAAAGGATCCTTGGAGGTCGGACGAGT-3' and antisense primer: 5'-CGGAGCCCCATGA AGTTAATCCA-3') were used to amplify a 630-bp fragment of corin cDNA between nucleotides 2475 and 3105. Oligonucleotide primers TFR1 (5'-GTCAATGTCCCAAACGTCACCAGA-3') and TFR2 (5'-ATTTCGGGAATGCTGAGAAAACAGACAGA-3'), derived from the human glyceraldehyde-3-phosphate dehydrogenase (GAPDH) gene, were used as an internal quantification control. PCR reactions were performed with a thermal cycler (Perkin-Elmer, model 480). PCR products were separated on 1% agarose gels and visualized by ethidium bromide staining.

*In Situ Hybridization--Mouse* adult heart and embryonic tissue sections were deparaffinized in xylene, rehydrated and fixed in 4% paraformaldehyde. The tissues were digested with proteinase K (20 mg/ml), then treated with triethanolamine/acetic anhydride and dehydrated. An 800-bp mouse corin cDNA fragment from the coding region was cloned into pCRII (Invitrogen) in two orientations to yield plasmids pM1 and pM41. The plasmids were linearized by HindIII digestion. Sense and antisense probes were synthesized using T7 RNA polymerase (T7/SP6 transcription kit, Boehringer Mannheim) and labeled with [³³P]UTP (Amersham). The hybridization was carried out as described (Jen et al., Dev. Dyn., 208:92-106, 1997). The slides were dehydrated and dipped in Kodak NTB-2 emulsion and exposed for 4 weeks in light-tight boxes at 4°C. Photographic development was carried out in a Kodak D-19 developer. The slides were stained with hemotoxin and eoisin and analyzed using both light- and dark-field optics of a Zeiss microscope.
*Fluorescent In Situ Hybridization (FISH) Analysis--*P1 phage clones containing the human corin gene were isolated by filter hybridization using a human corin cDNA as the probe. One clone was confirmed by DNA sequencing using a primer from human corin cDNA. The DNA fragment from this P1 phage was labeled with digoxigenin-dUTP. The labeled probe was combined with sheared human DNA and hybridized to metaphase chromosomes derived from PHA-stimulated peripheral blood lymphocytes in a solution containing 50% formamide, 10% dextran sulfate and 2 x SSC. Hybridization signals were detected by fluorescent-labeled antidigoxigenin antibodies and counter-staining with DAPI (4,6-diaminoidino-2-phenylindole). A total of 80 metaphase cells were analyzed of which 74 cells exhibited specific labeling.
*Homology Model of the Protease Domain of Corin--*A model of the corin protease domain (amino acids 802-1042) was built based on the structure of bovine chymotrypsinogen A at 1.8 A resolution (Wang et al., J. Mol. Biol., 185:595-624, 1985; Ponder and Richards, J. Mol. Biol., 1987), using the Homology program (Insight II, 1995, MSI, San Diego, CA). Rotamers were used for non-identical side-chain replacements (Ponder and Richards, J. Mol. Biol., 1987). Coordinates for the loop insertions were extracted from the Brookhaven protein data bank (Bernstein et al., Mol. Biol., 112:535-542, 1977). The model was refined by energy minimization using the AMBER force field (Discover 95.0), with a distance-dependent dielectric constant. The minimization used the steepest descents and conjugate gradient methods: first for the loops only where insertions and deletions occurred, then side-chains, and a final round of minimization keeping the Ca atoms fixed. The residues of corin (His843, Asp892 and Ser985) corresponding to the catalytic triad of the template structure were also held fixed.

### Biological activity of corin

Several different cell lines were produced by conventional methods to assay for the biological activity of corin. 293 cells were cotransfected with pro-ANF and corin expressing plasmids. Two additional cell lines were created by cotransfecting 293 cells with a pro-ANF expressing plasmid and a hepsin or prothrombin expressing plasmid. Only the cells expressing corin converted pro-ANF to ANF as demonstrated by Western blot using antibodies to ANF.
Another experiment was conducted in which conditioned media containing pro-ANF was contacted with a cells expressing recombinant corin polypeptide. Again, pro-ANF was converted to ANF. Control cells expressing hepsin or prothrombin had no effect on pro-ANF.

The nucleic acid fragments, cited above and in the figures, may be found in the Unigene, PubEST, and GenbBank databases as follows: Hs.62794 (AA126468 [1686098], AA126648 [1686206], AA625395 [2537780], AA046682 [1524579], AA249850 [1881137], and AA046793 [1524691]), and Hs.71798 (AA147031 [1716421]); Hs.121626 (AA771958), Hs.1657 (M69297), and Hs.47712 (AA203291), g1231787; g1312726; g1337948; and g942724.

## Claims

1. An isolated full-length human corin polypeptide comprising amino acid 1 to amino acid 1042 as set forth in SEQ ID NO:2.

2. The isolated full-length human corin polypeptide according to claim 1, coded for by the DNA sequence set forth in SEQ ID NO: 1.

3. An isolated nucleic acid comprising a nucleotide sequence coding for human full-length corin polypeptide comprising amino acid 1 to amino acid 1042 as set forth in SEQ ID NO:2.

4. The isolated nucleic acid according to claim 3, having the nucleotide sequence set forth in SEQ ID NO:1.

5. A vector comprising a nucleic acid according to claim 3.

6. A method of expressing in transformed host cells, a full-length human corin set forth in SEQ ID NO:2, coded for by a nucleic acid set forth in SEQ ID NO:1 comprising:
culturing transformed host cells containing a nucleic acid of SEQ ID NO:1 under conditions effective to express the polypeptide of SEQ ID NO:2; and
isolating the membrane fraction of said host cells comprising said polypeptide.

7. A method of identifying modulators of serine protease catalytic activity of a human corin polypeptide activity comprising:
reacting, in the presence of a test compound, a human corin polypeptide of claims 1 or 2, and chromogenic substrate for serine protease, under conditions effective for said polypeptide to cleave said substrate, which results in the appearance of a detectable color;
detecting said cleavage; and
identifying whether the test compound modulates said serine protease activity by comparing the amount of cleavage in the presence and absence of the test compound.

8. The method of claim 7, wherein said human corin polypeptide has the sequence of amino acid 1 to amino acid 1042 as set forth in SEQ ID NO:2.

9. The method of claim 7, wherein said human corin polypeptide is coded for by the nucleic acid sequence set forth in SEQ ID NO: 1.

10. The method of claim 7, wherein the substrate is pro-ANF.

## Patentansprüche

1. Isoliertes menschliches Corin-Polypeptid voller Länge, das Aminosäure 1 bis Aminosäure 1042, wie in Seq.-ID Nr. 2 gezeigt, umfasst.

2. Isoliertes menschliches Corin-Polypeptid voller Länge nach Anspruch 1, für das die in Seq.-ID Nr. 1 gezeigte DNA-Sequenz kodiert.

3. Isolierte Nucleinsäure, eine Nucleotidsequenz umfassend, die für ein menschliches Corin-Polypeptid voller Länge kodiert, das Aminosäure 1 bis Aminosäure 1042, wie in Seq.-ID Nr. 2 gezeigt, umfasst.

4. Isolierte Nucleinsäure nach Anspruch 3, die die in Seq.-ID Nr. 1 gezeigte Nucleotidsequenz aufweist.

5. Vektor, der eine Nucleinsäure nach Anspruch 3 umfasst.

6. Verfahren zur Expression eines menschlichen Corins voller Länge, wie in Seq.-ID Nr. 2 gezeigt, für das eine in Seq.-ID Nr. 1 gezeigte Nucleinsäure kodiert, in transformierten Wirtszellen, umfassend: das Kultivieren von transformierten Wirtszellen, die eine Nucleinsäure der Seq.-ID Nr. 1 umfassen, unter Bedingungen, die für die Expression des Polypeptids der Seq.-ID Nr. 2 geeignet sind, und das Isolieren der Membranfraktion dieser Wirtszellen, die das Polypeptid enthält.

7. Verfahren zur Identifikation von Modulatoren von katalytischer Serinproteasenaktivität von menschlicher Corin-Polypeptidaktivität, umfassend:
das Umsetzen eines menschlichen Corin-Polypeptids nach Anspruch 1 oder 2 und eines chromogenen Substrats für Serinprotease in Gegenwart einer Testverbindung unter Bedingungen, die dem Polypeptid die Spaltung des Substrats ermöglichen, was zum Auftreten einer nachweisbaren Farbe führt;
das Nachweisen der Spaltung; und
das Feststellen, ob die Testverbindung die Serinproteasenaktivität moduliert, indem das Ausmaß der Spaltung in Gegenwart und in Abwesenheit der Testverbindung verglichen wird.

8. Verfahren nach Anspruch 7, worin das menschliche Corin-Polypeptid die Sequenz von Aminosäure 1 bis Aminosäure 1042, wie in Seq.-ID Nr. 2 gezeigt, aufweist.

9. Verfahren nach Anspruch 7, worin für das menschliche Corin-Polypeptid die in Seq.-ID Nr. 1 gezeigte Nucleinsäuresequenz kodiert.

10. Verfahren nach Anspruch 7, worin das Substrat Pro-ANF ist.

## Revendications

1. Polypeptide humain corine isolé, de longueur complète, comprenant l'acide aminé 1 à l'acide aminé 1042, tel que donné en SEQ ID N°2.

2. Polypeptide humain corine isolé, de longueur complète, selon la revendication 1, codé par la séquence d'ADN donnée en SEQ ID N°1.

3. Acide nucléique isolé, comprenant une séquence des nucléotides codant le polypeptide humain corine, de longueur complète, comprenant l'acide aminé 1 à l'acide aminé 1042, tel que donné en SEQ ID N°2.

4. Acide nucléique isolé selon la revendication 3, ayant la séquence des nucléotides donnée en SEQ ID N°1.

5. Vecteur comprenant un acide nucléique selon la revendication 3.

6. Procédé d'expression dans des cellules hôtes transformées, d'une corine humaine de longueur complète, telle que donnée en SEQ ID N°2, codée par un acide nucléique donné en SEQ ID N°1, comprenant :
la culture des cellules hôtes transformées, contenant un acide nucléique de SEQ ID N°1, dans des conditions efficaces pour exprimer le polypeptide de SEQ ID N°2, et
l'isolement de la fraction membranaire desdites cellules hôtes comprenant ledit polypeptide.

7. Procédé d'identification de modulateurs de l'activité catalytique de protéase à sérine d'un polypeptide humain corine, comprenant :
la réaction, en présence d'un composé à tester, d'un polypeptide humain corine des revendications 1 ou 2, et du substrat chromogène pour la protéase à sérine, dans des conditions efficaces pour que ledit polypeptide clive ledit substrat, ce qui résulte en l'apparition d'une couleur détectable ;
la détection dudit clivage, et
l'identification de la modulation ou non par le composé à tester de ladite activité de protéase à sérine en comparant la quantité de clivage en présence et en absence du composé à tester.

8. Procédé selon la revendication 7, dans lequel ledit polypeptide humain corine a la séquence de l'acide aminé 1 à l'acide aminé 1042, tel que donné en SEQ ID N°2.

9. Procédé selon la revendication 7, dans lequel ledit polypeptide humain corine est codé par la séquence d'acide nucléique donnée en SEQ ID N°1.

10. Procédé selon la revendication 7, dans lequel le substrat est le pro-ANF.
